# EUROPEAN PATENT APPLICATION

(11) **EP 4 173 573 A1**
(43) Date of publication of application: **03.05.2023**
(21) Application number: 21852487.4
(22) Date of filing: 21.06.2021
(51) Int. Cl.: A61B 10/00, G01N 21/3554, A61B 5/00, A61B 5/16

(54) **BIOLOGICAL SENSOR AND BIOLOGICAL STATE DIFFERENTIATION METHOD**

(30) Priority: 07.08.2020 JP 2020134690
(71) Applicant: Sony Group Corporation, Minato-Ku, Tokyo, 108-0075 (JP)
(72) Inventor: ITO, Atsushi, Tokyo 108-0075 (JP)
(74) Representative: D Young & Co LLP
(86) International application number: PCT/JP2021/023292
(87) International publication number: WO 2022/030116

(57) **Abstract**

Provided is a biological sensor capable of easily determining a condition of a living body.

A biological sensor according to the present technology includes: an irradiation system that irradiates a living body with irradiation light including light having a wavelength within a wavelength band including a peak wavelength related to absorption of water; a light receiving system that individually receives the light having the wavelength within the wavelength band out of light emitted from the irradiation system and reflected from each of a plurality of sites of the living body; and a processing system that determines a condition of the living body on the basis of statistics related to signals of the respective sites output from the light receiving system.

## Description

### TECHNICAL FIELD

The technology according to the present disclosure (hereinafter also referred to as "the present technology") relates to a biological sensor and a biological condition determination method.

### BACKGROUND ART

Conventionally, an inspection method is known in which a living body is irradiated with light, and reflected light from the living body is imaged to visualize moisture distribution of the living body (for example, see Patent Document 1).

### CITATION LIST

### PATENT DOCUMENT

Patent Document 1: Japanese Patent Application Laid-Open No. H08-184555

### SUMMARY OF THE INVENTION

### PROBLEMS TO BE SOLVED BY THE INVENTION

However, it is difficult to easily determine a condition of a living body even if the conventional inspection method is used.

Therefore, a main object of the present technology is to provide a biological sensor and a biological condition determination method which enable easy determination of a condition of a living body.

### SOLUTIONS TO PROBLEMS

The present technology provides a biological sensor including:
an irradiation system that irradiates a living body with irradiation light including light having a wavelength within a wavelength band including a peak wavelength related to absorption of water;
a light receiving system that individually receives the light having the wavelength within the wavelength band out of light emitted from the irradiation system and reflected from each of a plurality of sites of the living body; and
a processing system that determines a condition of the living body on the basis of statistics related to signals of the respective sites output from the light receiving system.

The processing system may determine the condition of the living body by using a first distribution that is a distribution of a number of signals for each of signal values of the signals of the respective sites.

The processing system may determine the condition of the living body from a feature amount of the first distribution.

The feature amount of the first distribution may be the number of signals whose signal values are equal to or less than a reference value of the signals of the respective sites.

The reference value may be an average value of signal values of the signals of the respective sites.

The feature amount of the first distribution may be a median value, an average value, or a mode value of signal values of the signals of the respective sites.

The processing system may determine the condition of the living body by further using a second distribution that is a distribution of the number of signals for each of signal values of signals of the respective sites according to an inherent reflectance distribution of the plurality of sites.

The processing system may compare the feature amount of the first distribution with a feature amount of the second distribution to determine the condition of the living body.

The feature amount of the second distribution may be a feature amount corresponding to the feature amount of the first distribution.

The feature amount of the first distribution may be a proportion of the numbers of signals whose signal values respectively match a plurality of values among the signals of the respective sites in the first distribution, and the feature amount of the second distribution may be a proportion of the numbers of signals whose signal values respectively match the plurality of values among the signals of the respective sites in the second distribution.

The light receiving system may include: a light receiving element array including a plurality of light receiving elements respectively corresponding to the plurality of sites; and an optical system corresponding to the plurality of light receiving elements, and the optical system may guide the light having the wavelength within the wavelength band out of the light emitted from the irradiation system and reflected from each of the plurality of sites to the light receiving element corresponding to the site.

The irradiation light may include only the light having the wavelength within the wavelength band, and the optical system may include an optical member that guides the light having the wavelength within the wavelength band, which has been emitted from the irradiation system and reflected by a corresponding site among the sites, to a corresponding light receiving element among the light receiving elements.

The irradiation light may include the light having the wavelength within the wavelength band and light having a wavelength outside the wavelength band, and the optical system may include a wavelength selection filter that selectively passes the light having the wavelength within the wavelength band out of the light emitted from the irradiation system and reflected from each of the plurality of sites.

The optical system may include an optical member that guides the light having the wavelength within the wavelength band, which has passed through the wavelength selection filter, to a corresponding light receiving element among the light receiving elements.

The optical system may include an optical member that guides the light having the wavelength within the wavelength band to a corresponding light receiving element among the light receiving elements via the wavelength selection filter.

The irradiation system may include: a light source that emits the light having the wavelength within the wavelength band and the light having the wavelength outside the wavelength band; and a wavelength selection filter that selectively passes the light within the wavelength band out of the light within the wavelength band and the light outside the wavelength band emitted from the light source.

The optical system may include an optical member that guides the light having the wavelength within the wavelength band, which has passed through the wavelength selection filter and been reflected by a corresponding site among the sites, to a corresponding light receiving element among the light receiving elements.

The irradiation system may include at least one light source that is arranged between a pair of the light receiving elements adjacent to each other among the plurality of light receiving elements and emits light including the light having the wavelength within the wavelength band.

A light guide plate arranged between the optical system and the living body may be further provided, the irradiation system may include a light source that emits light including the light having the wavelength within the wavelength band, and the light guide plate may guide at least the light having the wavelength within the wavelength band out of the light emitted from the light source, and cause the light within the wavelength band to be incident on each of the plurality of sites.

The light guide plate may be transparent with respect to the light having the wavelength within the wavelength band.

The light guide plate may include a plurality of diffraction units respectively corresponding to the plurality of light receiving elements and arranged on a side of the optical system, and each of the plurality of diffraction units may diffract the incident light having the wavelength within the wavelength band toward a corresponding site among the sites.

A circularly polarizing plate arranged between the light receiving element array and the living body may be further provided.

The condition of the living body may be a mental condition estimated from a sweating state of each of the sites.

The present technology also provides a biological condition determination method including:
a step of irradiating a living body with irradiation light including light having a wavelength within a wavelength band including a peak wavelength related to absorption of water;
a step of outputting signals of a plurality of sites by individually receiving the light having the wavelength within the wavelength band out of light emitted from the irradiation system and reflected from each of a plurality of sites of the living body; and
a step of determining a condition of the living body on the basis of statistics related to the signals of the respective sites output in the outputting step.

### BRIEF DESCRIPTION OF DRAWINGS

Fig. 1 is a diagram schematically illustrating a configuration of a biological sensor according to Example 1 of an embodiment of the present technology.
Fig. 2 is a block diagram illustrating a functional example of the biological sensor according to Example 1 of the embodiment of the present technology.
Fig. 3 is a graph illustrating a first distribution and a second distribution.
Fig. 4 is a diagram illustrating average values of signal values of the first distribution and the second distribution in Fig. 3.
Fig. 5 is a diagram illustrating median values of the signal values of the first distribution and the second distribution in Fig. 3.
Fig. 6 is a diagram illustrating mode values of the signal values of the first distribution and the second distribution in Fig. 3.
Fig. 7 is a flowchart for describing biological condition determination processing 1.
Fig. 8 is a block diagram illustrating a functional example of a biological sensor according to a modified example of Example 1 of the embodiment of the present technology.
Fig. 9 is a flowchart for describing biological condition determination processing 2.
Fig. 10 is a diagram schematically illustrating a configuration of a biological sensor according to Example 2 of the embodiment of the present technology.
Fig. 11 is a diagram schematically illustrating a configuration of a biological sensor according to Example 3 of the embodiment of the present technology.
Fig. 12 is a diagram schematically illustrating a configuration of a biological sensor according to Example 4 of the embodiment of the present technology.
Fig. 13 is a diagram schematically illustrating a configuration of a biological sensor according to Example 5 of the embodiment of the present technology.
Fig. 14 is a diagram schematically illustrating a configuration of a biological sensor according to Example 6 of the embodiment of the present technology.
Fig. 15 is a diagram schematically illustrating a configuration of a biological sensor according to Example 7 of the embodiment of the present technology.
Fig. 16 is a diagram schematically illustrating a configuration of a biological sensor according to Example 8 of the embodiment of the present technology.
Fig. 17 is a diagram schematically illustrating a configuration of a biological sensor according to Example 9 of the embodiment of the present technology.
Fig. 18 is a diagram schematically illustrating a configuration of a biological sensor according to Example 10 of the embodiment of the present technology.
Fig. 19 is a diagram schematically illustrating a configuration of a biological sensor according to Example 11 of the embodiment of the present technology.
Fig. 20 is a diagram schematically illustrating a configuration of a biological sensor according to Example 12 of the embodiment of the present technology.
Fig. 21 is a diagram schematically illustrating a configuration of a biological sensor according to Example 13 of the embodiment of the present technology.
Fig. 22 is a diagram schematically illustrating a configuration of a biological sensor according to Example 14 of the embodiment of the present technology.
Fig. 23 is a diagram schematically illustrating a configuration of a biological sensor according to Example 15 of the embodiment of the present technology.
Fig. 24 is a diagram schematically illustrating a configuration of a biological sensor according to Example 16 of the embodiment of the present technology.
Fig. 25 is a diagram schematically illustrating a configuration of a biological sensor according to Example 17 of the embodiment of the present technology.
Fig. 26 is a diagram schematically illustrating a configuration of a biological sensor according to Example 18 of the embodiment of the present technology.
Fig. 27 is a diagram schematically illustrating a configuration of a biological sensor according to Example 19 of the embodiment of the present technology.
Fig. 28 is a diagram schematically illustrating a configuration of a biological sensor according to Example 20 of the embodiment of the present technology.
Fig. 29 is a diagram schematically illustrating a configuration of a biological sensor according to a modified example of Example 3 of the embodiment of the present technology.

### MODE FOR CARRYING OUT THE INVENTION

Hereinafter, a preferred embodiment of the present technology will be described in detail with reference to the accompanying drawings. Note that components having substantially the same functional configuration in the present specification and the drawings will be denoted by the same reference sign, and the redundant description thereof will be omitted. The embodiment to described hereinafter illustrates a representative embodiment of the present technology, and the scope of the present technology is not narrowly construed by the embodiment. In the present specification, each of a biological sensor and a biological condition determination method according to the present technology is only required to exhibit at least one effect even in a case where it is described that each of the biological sensor and the biological condition determination method according to the present technology exhibits a plurality of effects. The effects described in the present specification are merely examples and are not limited, and other effects may be present.

Furthermore, a description will be given in the following order.
1. Introduction
2. Biological Sensor According to Embodiment of Present Technology
3. Configuration of Biological Sensor According to Example 1 of Embodiment of Present Technology
4. Biological Condition Determination Processing 1
5. Effect of Biological Sensor and Effect of Biological Condition Determination Method According to Example 1 of Embodiment of Present Technology
6. Configuration of Biological Sensor According to Modified Example of Example 1 of Embodiment of Present Technology
7. Biological Condition Determination Processing 2
8. Effect of Biological Sensor According to Modified Example of Example 1 of Embodiment of Present Technology
9. Biological Sensors According to Examples 2 to 20 of Embodiment of Present Technology
10. Modified Example of Present Technology

### 1. <Introduction>

Conventionally, for example, sweat measurement devices have been widely used as means for measuring a mental condition of a person. As the sweat measurement device, for example, a ventilation capsule type measurement device that covers a skin with a capsule and observes a humidity change caused by sweating is known. Furthermore, as the sweat measurement device, for example, an electrodermal activity (EDA) measurement device that electrically measures a sweating phenomenon is also used. The EDA measurement device uses a phenomenon in which impedance and conductance of the skin change as a result of a mental activity of a person affecting an organ such as an eccrine gland of the skin. The EDA measurement device can be achieved by simpler mounting of pasting only two electrodes to the skin as compared with the ventilation capsule type measurement device, and thus, has been widely used.

However, in both the cases of the ventilation capsule type measurement device and the EDA measurement device, it is necessary to bring a measurement unit into contact with the skin, and there is a problem of causing discomfort at the time of wearing. Therefore, these sweat measurement devices are not generally widespread, and are often used in specialized institutions such as hospital. If the sweat measurement devices with such a restriction in use environment can be freely (easily) used without discomfort, it can be expected that a new product market for dynamically changing a service to be provided according to a mental condition of a person or the like will expand.

Therefore, as a result of intensive studies, the inventor has developed a non-contact biological sensor capable of easily determining a condition of a living body (for example, a human body) (for example, a mental condition estimated from a sweating state of the living body) as described below.

### 2. <Biological Sensor According to Embodiment of Present Technology>

Hereinafter, a biological sensor according to an embodiment of the present technology will be described.

The biological sensor according to the embodiment of the present technology is, for example, an optical sensor in which a measurement unit irradiates a surface (for example, skin) of a living body (for example, a human body) with light and receives reflected light to determine a condition (for example, a mental condition according to a sweating state of a person) of the living body.

More specifically, the biological sensor according to the embodiment is a non-contact biological sensor in which at least the measurement unit is used in a non-contact state with respect to the living body.

As use forms of the biological sensor according to the present technology, for example, a wide variety of forms are assumed such as a wearing type used by being worn on the living body, such as a wristband type, an earring type, a ring type, a necklace type, a stick-on type, and a supporter type, and a portable type used by being attached to the living body.

### 3. <Configuration of Biological Sensor According to Example 1 of Embodiment of Present Technology>

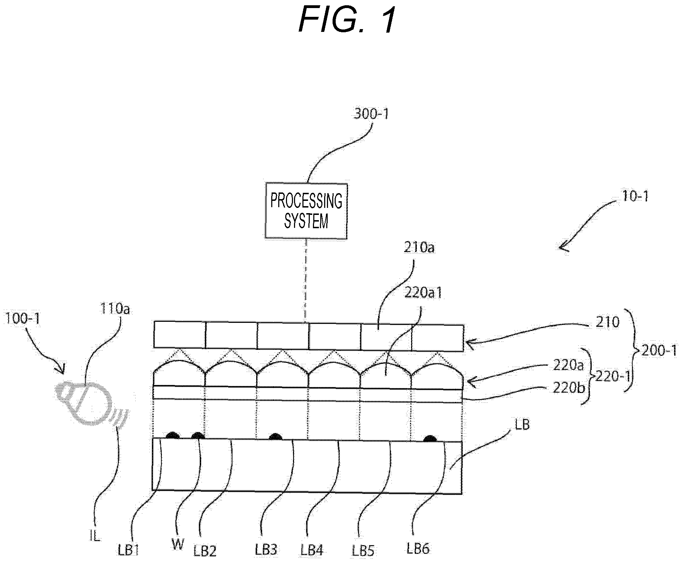

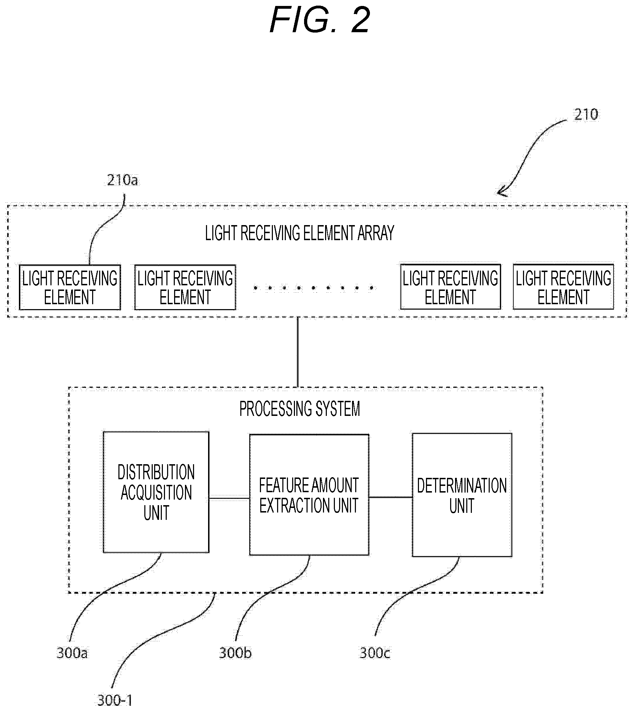

Fig. 1 is a diagram schematically illustrating a configuration of a biological sensor 10-1 according to Example 1 of the embodiment. In Fig. 1, a measurement unit (an irradiation system 100-1 and a light receiving system 200-1 to be described later) of the biological sensor 10-1 are illustrated in a cross-sectional view. Fig. 2 is a block diagram illustrating Functional Example 1 of the biological sensor 10-1 according to Example 1 of the embodiment.

As illustrated in Fig. 1, the biological sensor 10-1 of Example 1 includes the irradiation system 100-1, the light receiving system 200-1, and a processing system 300-1.

The irradiation system 100-1 and the light receiving system 200-1 are integrated, for example. The processing system 300-1 may be integrated with the irradiation system 100-1 and the light receiving system 200-1, or may be separately provided.

As illustrated in Fig. 1, the biological sensor 10-1 is used in a state where the measurement unit including the irradiation system 100-1 and a light receiving system 200-1 is not in contact with (for example, in proximity to) a living body LB.

Note that the living body LB includes, for example, not only a human body but also a body of an animal or the like other than a human.

### (Irradiation System)

The irradiation system 100-1 irradiates the living body LB (specifically, a surface of the living body LB) with irradiation light IL including light having a wavelength within a wavelength band WB including a peak wavelength λ_{P} related to absorption of water.

The peak wavelength λ_{P} related to the absorption of water is a wavelength that takes a peak (maximum value) in a graph illustrating an absorption spectrum of water (horizontal axis: wavelength, vertical axis: absorptivity).

As an example, the wavelength band WB includes a plurality of (for example, two) wavelength bands existing in a wavelength range of 1300 nm to 2100 nm and having relatively high water absorbability.

One of the two wavelength bands WB is a first wavelength band WB1 including a peak wavelength λ_{P1} (for example, 1450 nm). The first wavelength band WB1 is, for example, preferably λ_{P1} - 100 nm ≤ WB1 ≤ λ_{P1} + 100 nm, and more preferably λ_{P1} - 50 nm ≤ WB1 ≤ λ_{P1} + 50 nm.

The other of the two wavelength bands WB is a second wavelength band WB2 including a peak wavelength λ_{P2} (for example, 1940 nm). The second wavelength band WB2 is, for example, preferably λ_{P2} < 100 nm ≤ WB2 ≤ λ_{P2} + 100 nm, and more preferably λ_{P2} - 50 nm ≤ WB2 ≤ λ_{P2} + 50 nm.

The irradiation system 100-1 includes a light source 110a. The light source 110a is, for example, a halogen lamp, a light emitting diode (LED), a laser, or the like. Light emitted from the light source 110a may be visible light or invisible light, but is preferably the invisible light (for example, infrared light).

As an example, as illustrated in Fig. 1, the light source 110a is positioned with respect to the light receiving system 200-1 so as to emit light from a direction inclined with respect to the surface of the living body LB when the light receiving system 200-1 is opposite to the surface of the living body LB.

The light source 110a emits light having a wavelength within the wavelength band WB and light having a wavelength outside the wavelength band WB. Here, the light emitted from the light source 110a is the irradiation light IL. That is, the irradiation light IL includes light having a wavelength within the wavelength band WB and light having a wavelength outside the wavelength band WB.

The irradiation light IL is only required to include light having a wavelength within at least one of the first wavelength band WB1 or the second wavelength band WB2.

More specifically, the irradiation light IL may include light having at least one wavelength within the first wavelength band WB1, may include light having at least one wavelength within the second wavelength band WB2, or may include light having at least one wavelength within the first wavelength band WB1 and light having at least one wavelength within the second wavelength band WB2.

### (Light Receiving System)

The light receiving system 200-1 individually receives light having a wavelength within the wavelength band WB out of the light emitted from the irradiation system 100-1 and reflected from each of a plurality of sites of the living body LB.

The light receiving system 200-1 includes a light receiving element array 210 including a plurality of (for example) light receiving elements 210a and an optical system 220-1 corresponding to the plurality of light receiving elements 210a.

As an example, the plurality of light receiving elements 210a is arranged in a two-dimensional array.

The plurality of light receiving elements 210a corresponds to a plurality of sites (for example, LB1 to LB6) of the living body LB, respectively.

Each of the light receiving elements 210a has sensitivity in a wavelength range of 1400 nm to 2000 nm, for example.

Each of the light receiving elements 210a is, for example, a pixel sensor including a material such as InGaAs, PbSe, or InSb. That is, the light receiving element array 210 is an image sensor (area sensor) including a plurality of the pixel sensors.

For example, a pixel sensor including relatively inexpensive InGaAs has sensitivity to a wavelength of approximately 1700 nm or less. In this case, the irradiation light IL is only required to include light having a wavelength within the first wavelength band WB1.

Each of the light receiving elements 210a photoelectrically converts incident light and outputs an electric signal.

As the light receiving element 210a, for example, a photodiode (PD), a phototransistor, or the like is used.

The light receiving element is also called, for example, a "light receiver", a "photodetector", or the like.

### (Optical System)

The optical system 220-1 guides light having a wavelength within the wavelength band WB out of the light emitted from the irradiation system 100-1 and reflected from each of the plurality of sites of the living body LB to the light receiving element 210a corresponding to the site.

The optical system 220-1 includes a lens array 220a (optical member) including a plurality of light receiving lenses 220a1 and a wavelength selection filter 220b.

In the optical system 220-1, the lens array 220a and the wavelength selection filter 220b are arranged adjacent to each other such that the lens array 220a is arranged on the light receiving element array 210 side and the wavelength selection filter 220b is arranged on the living body LB side.

The wavelength selection filter 220b selectively passes the light having the wavelength within the wavelength band WB out of the light emitted from the irradiation system 100-1 and reflected from each of the plurality of sites of the living body LB. The wavelength selection filter 220b is, for example, a band-pass filter having the wavelength band WB as a pass band.

The lens array 220a is arranged on a side opposite to the living body LB side of the wavelength selection filter 220b. The lens array 220a guides the light having the wavelength within the wavelength band WB, which has passed through the wavelength selection filter 220b, to the corresponding light receiving element 210a.

As an example, the plurality of light receiving lenses 220a1 of the lens array 220a is arranged in the two-dimensional array so as to face the plurality of corresponding light receiving elements 210a.

Each of the light receiving lenses 220a1 is a lens that is convex toward the corresponding light receiving element 210a, and collects (preferably forms an image) light from a corresponding site of the living body LB, which has passed through the wavelength selection filter 220b, on the corresponding light receiving element 210a.

That is, only light from the corresponding site of the living body LB is incident on each of the light receiving elements 210a by the light collecting action of the corresponding light receiving lens 220a1. Therefore, incidence (crosstalk) of light from a non-corresponding site of the living body LB onto each of the light receiving elements 210a is suppressed.

### (Mechanism of Non-Contact Sweat Measurement)

Meanwhile, as is well known, sweat does not uniformly come out from a surface of a living body, but discretely comes out from an organ called a sweat gland on the surface of the living body. Therefore, among the plurality of sites of the surface of the living body, a site having the sweat gland has a substantially lower reflectance than a site having no sweat gland due to absorbance by water (sweat). Such a decrease in reflectance increases in a site having a larger number of sweat glands. As the reflectance of each site of the living body decreases, the amount of reflected light from the site decreases, and the amount of received light in a light receiving element corresponding to the site decreases.

More specifically, there are two sweat glands at the site LB1 of the living body LB on the leftmost side when facing the paper surface in Fig. 1 as an example, and sweat W comes out from the two sweat glands. In this case, the amount of received light in the light receiving element 210a corresponding to the site (a signal value of an output signal) greatly decreases as compared with a case where no sweat W comes out from the two sweat glands.

In Fig. 1, there are no sweat gland at the second site LB2, the fourth site LB4, and the fifth site LB5 counted from the leftmost site of the living body LB when facing the paper surface, and the amount of received light in the light receiving element 210a corresponding to each of these sites does not change.

In Fig. 1, one sweat gland is present at each of the third site LB3 and the sixth site LB6 from the leftmost site of the living body LB when facing the paper surface, and the sweat W comes out from each of the sweat glands. In this case, the amount of received light in the light receiving element 210a corresponding to each of these sites decreases as compared with a case where no sweat W comes out from each of the sweat glands.

As a result, it is estimated that the decrease in the amount of received light in the light receiving element 210a corresponding to LB1 is greater than the decrease in the amount of received light in the light receiving element 210a corresponding to each of LB3 and LB6.

### (Problem of Non-Contact Sweat Measurement)

Although it is possible to perform sweat measurement for each of the sites of the living body LB in a non-contact manner as described above, it is difficult to compare sweating states of the plurality of corresponding sites even if the amounts of received light in the plurality of light receiving elements 210a are simply compared. This is because it is difficult to keep relative positions of the plurality of light receiving elements 210a and the living body LB in a direction along the surface of the living body LB strictly coincident with each other. In general, a density of human sweat glands is 130 to 600 sweat glands/cm², and an average sweat gland interval is calculated to be 0.4 to 0.8 mm. Thus, even if the relative position deviates by about 1 mm, the correspondence relationship between the plurality of sites of the living body LB and the plurality of light receiving elements 210a changes, so that the sweating states of the sites corresponding to the light receiving elements 210a on a one-to-one basis are not acquired even if the amount of received light of each of the light receiving elements 210a is acquired.

Therefore, if statistics related to the signal of each of the sites of the living body LB output from the light receiving system 200-1 are obtained, a sweating state (sweating tendency) as a whole of the plurality of sites of the living body LB can be known, and a condition (for example, a mental condition) of the living body LB can be determined from the sweating state.

In addition, the biological sensor according to the embodiment utilizes the fact that sweat discretely comes out at sweat gland positions in a minute sweating phenomenon such as mental sweating. At this time, measurement is performed by the plurality of light receiving elements arranged in the array, but the amount of sweat is measured using a statistic amount of all outputs of the plurality of light receiving elements instead of observing output values of the individual light receiving elements.

For example, in a case where a skin in which sweating does not occur is observed, when a histogram of outputs from the plurality of light receiving elements is created, an output distribution having a substantially normal distribution shape according to an average reflectance distribution of the skin is obtained. On the other hand, when sweating occurs, a reflectance of a light receiving element that receives reflected light from a site having a sweat gland decreases due to moisture of sweat. Therefore, the centroid of the reflectance distribution moves from the substantially normal distribution to the lower output side. When sweating further progresses, an output further shifts to the lower output side, and the outputs of all the light receiving elements become zero finally when the skin is dripping with sweat.

### (Processing System)

The processing system 300-1 determines a condition of the living body LB on the basis of the statistics related to the signals of each of sites of the living body LB output from the light receiving system 200-1.

The condition of the living body LB is, for example, a mental condition estimated from a sweating state of each of the sites of the living body LB.

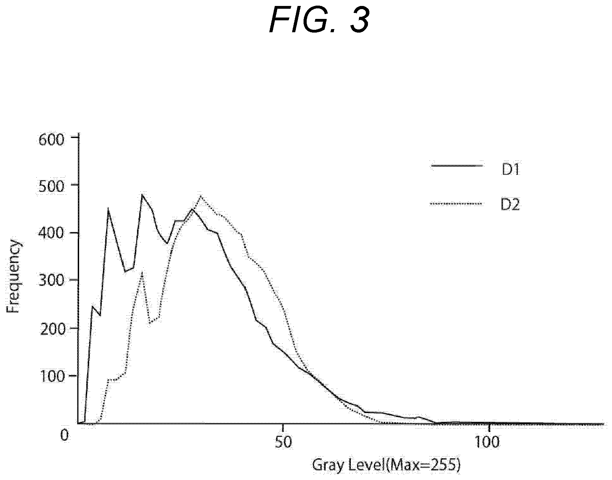

As an example, the processing system 300-1 determines the condition of the living body using a first distribution D1 (see Fig. 3) that is a distribution of the number of signals for each of signal values of the signals of the respective sites of the living body LB. In Fig. 3, the horizontal axis represents the signal value, and the vertical axis represents the number of signals.

Here, as illustrated in Fig. 3, the distribution of the number of signals for each signal value of the signals of each of the plurality of sites of the living body LB when it is assumed that the living body LB is not sweating is a substantially normal distribution (hereinafter referred to as a "second distribution D2") according to an inherent reflectance distribution of the plurality of sites.

On the other hand, since the first distribution D1 is the distribution of the number of signals for each of the signal values of the signals of the respective sites of the living body LB when the living body LB is sweating as described above, the first distribution D1 becomes a distribution in which the absorptivity (absorbance) due to the sweat W adhering to the site is added to the inherent reflectance of the site, that is, a distribution deviating to a side where the signal value becomes lower than that of the second distribution. In this case, a feature amount of the first distribution is an index indicating a sweating state of the living body LB.

Therefore, the processing system 300-1 can determine the condition of the living body LB from the feature amount of the first distribution (for example, the mental condition estimated from the sweating state of the living body LB).

More specifically, in Fig. 3, the second distribution D2 is an output distribution having a substantially normal distribution centered on a signal value of about 30. On the other hand, in the first distribution D1, an average of signal values decreases to about 20, and the centroid deviates to the side where the signal value becomes lower than that of the second distribution D2.

The presence or absence of the sweating phenomenon and the amount of sweating can be obtained without performing complicated processing by statistically processing all the outputs without capturing the outputs of the individual light receiving elements. Since such statistical processing is performed, a substantially normal distribution is obtained in a case where there is no sweating even if there is an external factor such as a case where external light is incident or a case where a measurement position of the skin deviates, and it can be seen that sweating occurs by an amount corresponding to the amount of displacement in a case where the distribution is displaced to the lower output side. Therefore, it is possible to continuously measure the amount of sweat regardless of the external factor by performing the statistical processing using the plurality of light receiving elements.

As illustrated in Fig. 2, the processing system 300-1 includes a distribution acquisition unit 300a, a feature amount extraction unit 300b, and a determination unit 300c. The processing system 300-1 is achieved, for example, by hardware such as a CPU and a chip set.

The distribution acquisition unit 300a acquires the first distribution D1.

The feature amount extraction unit 300b extracts a feature amount from the first distribution D1.

The feature amount of the first distribution D1 may be anything as long as the feature amount indicates a deviation of the first distribution from the second distribution such as a distortion (kurtosis or skewness), a variance, or a standard deviation of the first distribution D1, for example. As a difference between the feature amount of the first distribution and a corresponding feature amount of the second distribution is larger, the amount of sweat of the living body LB is larger, and it can be estimated that the living body LB is in, for example, a tenser state, a more stressed state, a more disturbed autonomic nerve state, or the like.

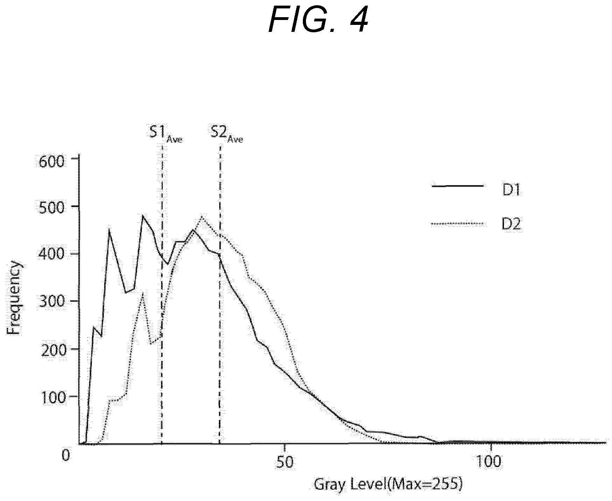

For example, as illustrated in Fig. 4, the feature amount of the first distribution D1 may be an average value S1_{Ave} of signal values of signals of the respective sites of the living body LB in the first distribution D1. The average value S1_{Ave} is smaller than an average value S2_{Ave} of signal values of signals of the respective sites of the living body LB in the second distribution D2, and it is estimated that the amount of sweat in the living body LB is larger as the value is smaller.

For example, the feature amount of the first distribution D1 may be the number of signals having signal values equal to or less than, for example, the average value S1_{AVe} (reference value) of the signals of the respective sites of the living body LB in the first distribution D1. The number of signals is larger than the number of signals having signal values equal to or less than the average value S2_{AVe} at each of the sites of the living body LB in the second distribution D2, and is estimated that the amount of sweat in the living body LB is larger as the value is larger. Note that the reference value is not limited to the average value S1_{AVe} of the signal values of the signals of the respective sites of the living body LB in the first distribution D1, and is only required to be a value less than the maximum value of the signal values.

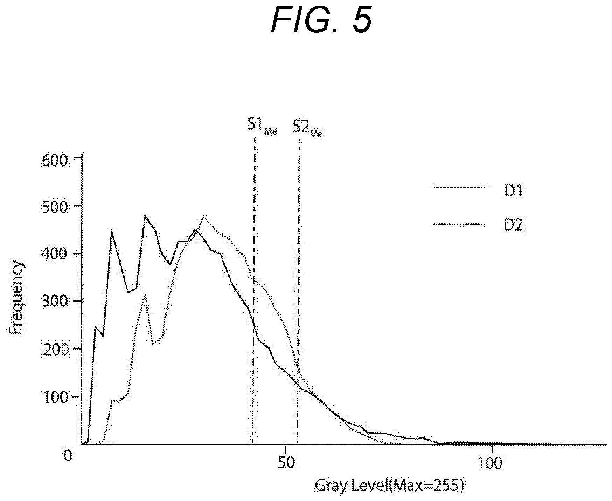

For example, as illustrated in Fig. 5, the feature amount of the first distribution D1 may be a median value S1_{Me} of signal values of signals of the respective sites of the living body LB. The median value S1_{Me} is smaller than a median value S2_{Me} of signal values of signals of the respective sites of the living body LB in the second distribution D2, and it is estimated that the amount of sweat in the living body LB is larger as the value is smaller.

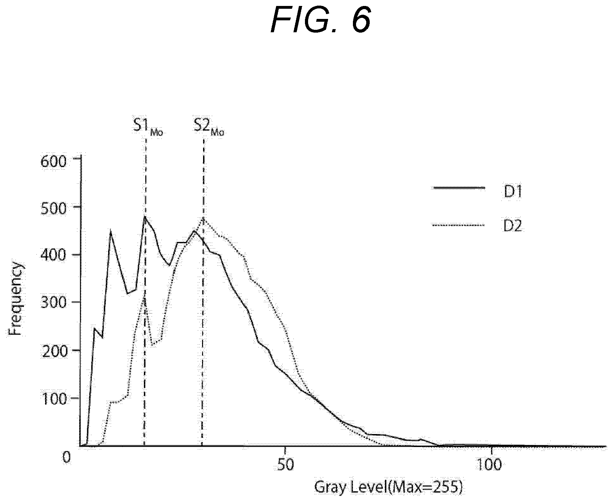

For example, as illustrated in Fig. 6, the feature amount of the first distribution D1 may be a mode value S1_{Mo} (signal value at which the number of signals in the first distribution peaks) of signal values of signals of the respective sites of the living body LB. The mode value S1_{Mo} is smaller than a mode value S2_{Mo} (signal value at which the number of signals in the second distribution peaks) of signal values of signals of the respective sites of the living body LB in the second distribution D2, and it is estimated that the amount of sweat in the living body LB is larger as the value is smaller.

### 4. <Biological Condition Determination Processing 1>

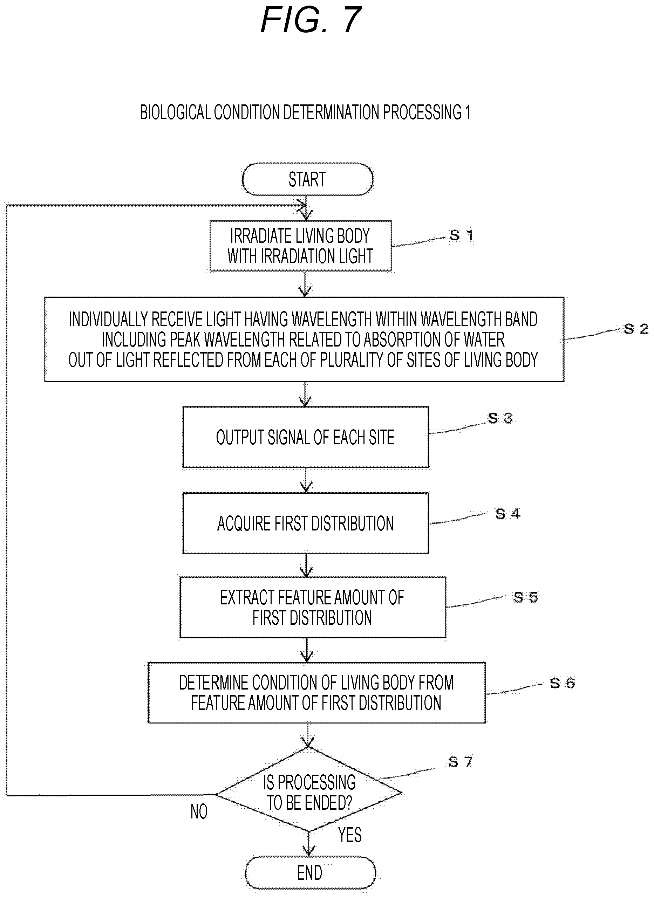

Hereinafter, biological condition determination processing 1 performed using the biological sensor 10-1 will be described with reference to a flowchart of Fig. 7. The biological condition determination processing 1 is an example of the biological condition determination method according to the present technology. The biological condition determination processing 1 is started, for example, when the biological sensor 10-1 in a state in which the measurement unit is opposite and in proximity to the living body LB is powered on.

In the first step S1, the irradiation system 100-1 irradiates the living body LB with the irradiation light IL. Specifically, the irradiation system 100-1 causes the light source 110a to emit light, and irradiates the living body LB with the irradiation light IL including light having a wavelength within the wavelength band WB and light having a wavelength outside the wavelength band WB.

In the next step S2, the light receiving system 200-1 individually receives light having a wavelength within the wavelength band WB including the peak wavelength λ_{P} related to absorption of water out of light reflected from each of the plurality of sites of the living body LB. Specifically, the light within the wavelength band WB out of the light (irradiation light IL) applied to and reflected from each of the plurality of sites of the living body LB passes through the wavelength selection filter 220b and is collected on the corresponding light receiving element 210a by the corresponding light receiving lens 220a1.

In the next step S3, the light receiving system 200-1 outputs signals of the respective sites of the living body LB. Specifically, each of the plurality of light receiving elements 210a photoelectrically converts the received light and outputs an electric signal corresponding to the amount of the received light.

In the next step S4, the processing system 300-1 acquires the first distribution D1. Specifically, the distribution acquisition unit 300a acquires a distribution of the number of signals for each of signal values of the signals of the respective sites of the living body LB (see Fig. 3).

In the next step S5, the processing system 300-1 extracts a feature amount of the first distribution D1. Specifically, the feature amount extraction unit 300b extracts, from the first distribution D1, for example, the average value S1_{Ave}, the number of signals whose signal values are equal to or less than the average value S1_{Ave}, the median value S1_{Me}, the mode value S1_{Mo}, and the like.

In the next step S6, the processing system 300-1 determines a condition of the living body LB from the feature amount of the first distribution D1.

For example, in a case where the feature amount of the first distribution D1 is the average value S1_{Ave}, the determination unit 300c determines that the living body LB is in the tenser state as the value is smaller (as the amount of sweat of the living body LB is larger), and determines that the living body LB is in the more relaxed state as the value is larger (as the amount of sweat of the living body LB is smaller).

For example, in a case where the feature amount of the first distribution D1 is the number of signals whose signal values are equal to or less than the average value S1_{Ave}, the determination unit 300c determines that the living body LB is in the tenser state as the number of signals is larger (as the amount of sweat of the living body LB is larger), and determines that the living body LB is in the more relaxed state as the number of signals is smaller (as the amount of sweat of the living body LB is smaller).

For example, in a case where the feature amount of the first distribution is the median value S1_{Me}, the determination unit 300c determines that the living body LB is in the tenser state as the value is smaller (as the amount of sweat of the living body LB is larger), and determines that the living body LB is in the more relaxed state as the value is larger (as the amount of sweat of the living body LB is smaller).

For example, in a case where the feature amount of the first distribution is the mode value S1_{Mo}, the determination unit 300c determines that the living body LB is in the tenser state as the value is smaller (as the amount of sweat of the living body LB is larger), and determines that the living body LB is in the more relaxed state as the value is larger (as the amount of sweat of the living body LB is smaller).

Note that the determination unit 300c may output a determination result as described above (for example, the mental condition of the living body LB according to the magnitude of the feature amount of the first distribution) in evaluation stages. For example, determination results may be output in ten stages in which a case where the living body LB is in the most relaxed state is set to 1 and a case where the living body LB is in the tense state is set to 10.

In the final step S7, the processing system 300-1 determines whether or not to end the processing. Specifically, the processing system 300-1 ends the processing, for example, when the biological sensor 10-1 is powered off. The flow ends if the determination in step S7 is positive, and returns to step S1 if the determination is negative.

### 5. <Effects of Biological Sensor and Biological Condition Determination Method According to Example 1 of Embodiment of Present Technology>

The biological sensor 10-1 according to Example 1 includes: the irradiation system 100-1 that irradiates the living body LB with the irradiation light IL including light having a wavelength within the wavelength band WB including the peak wavelength λ_{P} related to absorption of water; the light receiving system 200-1 that individually receives the light having the wavelength within the wavelength band WB out of the light emitted from the irradiation system 100-1 and reflected from each of the plurality of sites of the living body LB; and the processing system 300-1 that determines a condition of the living body LB on the basis of statistics related to signals of each of sites output from the light receiving system 200-1.

Therefore, the condition of the living body LB can be determined from the statistics of signal values corresponding to substantial reflectances (reflectance accurately reflecting presence or absence and amount of the sweat W) in the plurality of sites of the living body LB, and thus, the condition of the living body LB can be easily determined.

As a result, it is possible to provide a biological sensor capable of easily determining the condition of the living body LB according to the biological sensor 10-1.

Note that light having a wavelength outside the wavelength band WB has relatively low water absorbability, and thus, even if the light outside the wavelength band WB out of the light reflected by the living body LB is received by the light receiving element array, the light reception result thereof does not accurately reflect the presence or absence and amount of the sweat W.

On the other hand, in a method of obtaining the output of each of the light receiving elements as described above, a relative position between the biological sensor and the living body slightly deviates so that the correspondence relationship between the plurality of light receiving elements and the plurality of sites of the living body changes. As a countermeasure thereof, a method of regarding the output of the light receiving element array as an image to perform tracking, tracking signals of each sweat gland, and acquiring continuous values can be considered. However, even if such a method is used, a large amount of calculation is required, it is difficult to achieve the biological sensor capable of easily determining the condition of the living body.

Furthermore, as another method, there is also a method of defining an average value of all measurement values of the light receiving element array as the amount of sweat. In this case, the output of each of the light receiving elements is not important, and it can be considered that the amount of sweat increases if the overall reflectance simply decreases. With this configuration, the light receiving elements are not necessarily arranged in an array, and a single light receiving element may be used. In such a case, however, it is difficult to perform continuous measurement, for example, in a case where the entire sensor floats from the skin and external light enters when the living body moves, or a case where the entire sensor laterally moves so that the average reflectance of the skin changes.

In a case where it is desired to know a mental condition of a person, not only a restful case in hospital, a laboratory, or the like is assumed, but also there is a demand for being worn on the person as a wearable device to measure a sweating state in a daily life environment, and thus, sufficient countermeasures are required for the entry of external light and the like, and further improvement is required.

The biological sensor according to the present technology does not perform the tracking and can stably implement the sweat measurement with similar simplicity with the use of the average value of all the measurement values described above.

The processing system 300-1 determines the condition of the living body LB using the first distribution that is the distribution of the number of signals for each of the signal values of the signals of the respective sites of the living body LB. Therefore, it is possible to more easily determine the condition of the living body LB.

The processing system 300-1 preferably determines the condition of the living body LB from the feature amount of the first distribution D1. Therefore, the condition of the living body LB can be more easily and accurately determined.

The feature amount of the first distribution D1 may be the average value S1_{Ave}, a median value S_{Me}, or a mode value S_{Mo} of signal values of the signals of the respective sites of the living body LB. Therefore, for example, an index of the amount of sweat can be calculated by processing extremely easier than the calculation of skewness.

The feature amount of the first distribution D1 may be the number of signals whose signal values are equal to or less than the reference value of the signals of the respective sites of the living body LB. Therefore, for example, an index of the amount of sweat can be calculated by processing extremely easier than the calculation of skewness. In this case, the reference value may be, for example, the average value S1_{Ave} of the signal values of the signals of the respective sites of the living body LB, or may be another value. In a case where the reference value is the average value S1_{Ave}, an appropriate determination result can be obtained extremely easily.

The light receiving system 200-1 includes: the light receiving element array 210 including the plurality of light receiving elements 210a respectively corresponding to the plurality of sites of the living body LB; and the optical system 220-1 corresponding to the plurality of light receiving elements 210a. The optical system 220-1 guides the light having the wavelength within the wavelength band WB out of the light emitted from the irradiation system 100-1 and reflected from each of the plurality of sites of the living body LB to the light receiving element 210a corresponding to the site. Therefore, the light having the wavelength within the wavelength band WB out of the light reflected from each of the sites of the living body LB can be reliably guided to the light receiving element 210a corresponding to the site.

The irradiation light IL includes light having a wavelength within the wavelength band WB and light having a wavelength outside the wavelength band WB, and the optical system 220-1 includes the wavelength selection filter 220b that selectively passes the light having the wavelength within the wavelength band WB out of the light emitted from the irradiation system 100-1 and reflected from each of the plurality of sites. Therefore, it is possible to shield the light having the wavelength outside the wavelength band WB having relatively low water absorbability out of the light reflected at each of the sites of the living body LB.

The optical system 220-1 includes the light receiving lens 220a1 that guides the light having the wavelength within the wavelength band WB that has passed through the wavelength selection filter 220b to the corresponding light receiving element 210a. Therefore, the light having the wavelength within the wavelength band WB that has passed through the wavelength selection filter 220b out of the light reflected from each of the sites of the living body LB can be reliably guided to the corresponding light receiving element 210a.

The condition of the living body LB is, for example, a mental condition estimated from a sweating state of each of the sites of the living body LB. Therefore, it is possible to provide the biological sensor 10-1 that can easily determine the mental condition of the living body LB.

The biological condition determination method (for example, the biological condition determination processing 1) using the biological sensor 10-1 according to Example 1 of an embodiment of the present technology includes: a step of irradiating the living body LB with the irradiation light IL including light having a wavelength within the wavelength band WB including the peak wavelength λ_{P} related to absorption of water; a step of outputting signals of a plurality of sites by individually receiving the light having the wavelength within the wavelength band WB out of light emitted from the irradiation system 100-1 and reflected from each of the sites of the living body LB; and a step of determining a condition of the living body LB on the basis of statistics related to the signals of the respective sites output in the outputting step.

Therefore, the condition of the living body LB can be determined from the statistics of signal values corresponding to substantial reflectances (reflectance also reflecting the influence of the sweat W) in the plurality of sites, and thus, the condition of the living body LB can be easily determined.

As a result, the condition of the living body LB can be easily determined according to the biological condition determination method using the biological sensor 10-1.

### 6. <Configuration of Biological Sensor According to Modified Example of Example 1 of Embodiment of Present Technology>

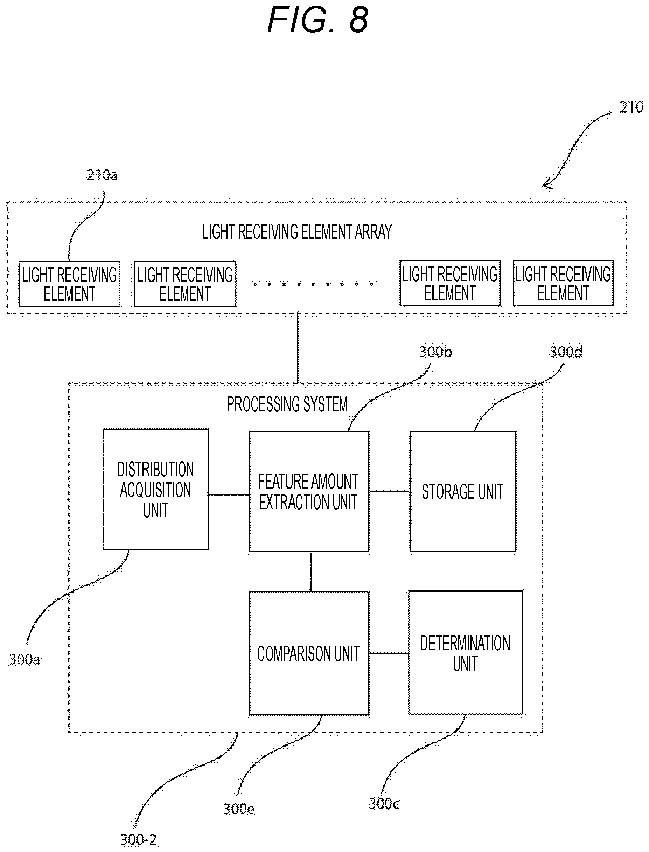

As illustrated in Fig. 8, a biological sensor according to a modified example of Example 1 has a configuration (see Fig. 1) similar to that of the biological sensor 10-1 according to Example 1 except that the configuration of the processing system is different.

A processing system 300-2 of the biological sensor according to the modified example includes a storage unit 300d and a comparison unit 300e in addition to the configuration of the processing system 300-1 (see Fig. 2).

The storage unit 300d stores the second distribution D2 acquired in advance. The second distribution D2 is acquired in advance, for example, by performing measurement using the biological sensor according to the modified example on the living body LB in a restful and relaxed state (state without sweating).

The storage unit 300d is achieved by, for example, a ROM, a RAM, a flash memory, a hard disk, or the like.

In the processing system 300-2, the feature amount extraction unit 300b extracts a feature amount of the first distribution D1 acquired by the distribution acquisition unit 300a, and extracts a feature amount of the second distribution D2 stored in the storage unit 300d ((the same type of) feature amount corresponding to the feature amount of the first distribution D1).

The comparison unit 300e compares the feature amounts of the first and second distributions D1 and D2 extracted by the feature amount extraction unit 300b. The comparison unit 300e is implemented by, for example, a CPU or the like.

In the processing system 300-2, the determination unit 300c determines a condition of the living body LB according to a comparison result in the comparison unit 300e.

### 7. <Biological Condition Determination Processing 2>

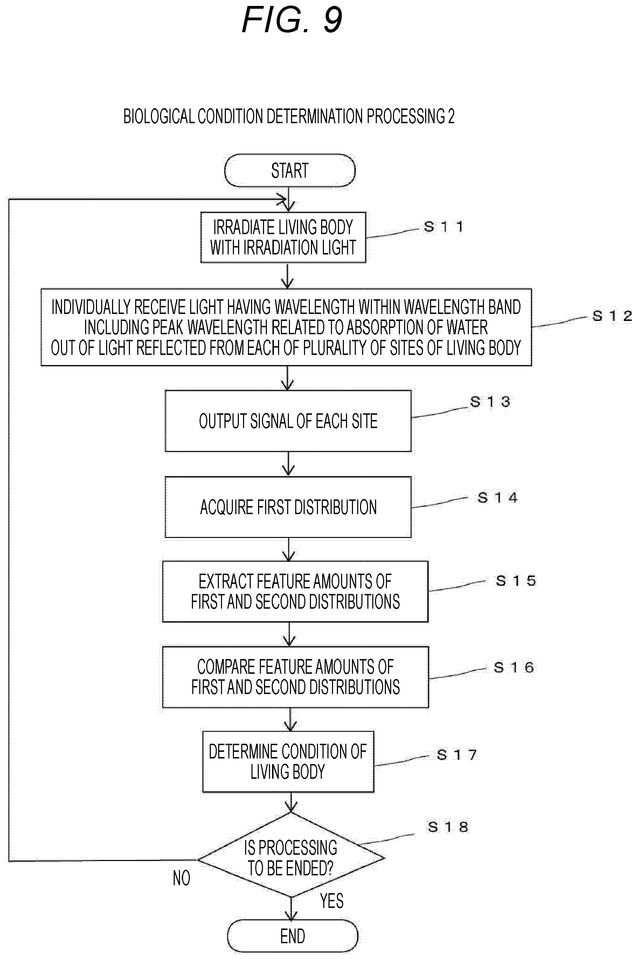

Hereinafter, biological condition determination processing 2 performed using the biological sensor according to the modified example of Example 1 will be described with reference to a flowchart of Fig. 9. The biological condition determination processing 2 is an example of the biological condition determination method according to the present technology. The biological condition determination processing 1 is started, for example, when the biological sensor in a state in which a measurement unit is opposite and in proximity to the living body LB is powered on.

In the first step S11, the irradiation system 100-1 irradiates the living body LB with the irradiation light IL. Specifically, the irradiation system 100-1 causes the light source 110a to emit light, and irradiates the living body LB with the irradiation light IL including light having a wavelength within the wavelength band WB and light having a wavelength outside the wavelength band WB.

In the next step S12, the light receiving system 200-1 individually receives light having a wavelength within the wavelength band WB including the peak wavelength λ_{P} related to absorption of water out of light reflected from each of a plurality of sites of the living body LB. Specifically, the light within the wavelength band WB out of the light (irradiation light IL) applied to and reflected from each of the plurality of sites of the living body LB passes through the wavelength selection filter 220b and is collected on the corresponding light receiving element 210a by the corresponding light receiving lens 220a1.

In the next step S13, the light receiving system 200-1 outputs signals of the respective sites of the living body LB. Specifically, each of the plurality of light receiving elements 210a photoelectrically converts the received light and outputs an electric signal corresponding to the amount of the received light to the processing system 300-2.

In the next step S14, the processing system 300-2 acquires the first distribution D1. Specifically, the distribution acquisition unit 300a acquires a distribution (see Fig. 3) of the number of signals for each of signal values of the signals of the respective sites of the living body LB from the input signals of the respective sites of the living body LB, and outputs an acquisition result to the feature amount extraction unit 300b.

In the next step S15, the processing system 300-2 extracts feature amounts of the first and second distributions D1 and D2. Specifically, the feature amount extraction unit 300b extracts the feature amount of the input first distribution D1 and the feature amount of the second distribution D2 stored in the storage unit 300d, and outputs the feature amounts of the first and second distributions D1 and D2 to the comparison unit 300e.

For example, the feature amount extraction unit 300b extracts the average value S1_{Ave} from the first distribution D1 and extracts the average value S2_{Ave} from the second distribution D2 (see Fig. 4).

For example, the feature amount extraction unit 300b extracts the number of signals whose signal values are equal to or less than a reference value (for example, the average value S1_{Ave}) from the first distribution D1, and extracts the number of signals whose signal values are equal to or less than a reference value (for example, the average value S2_{Ave}) from the second distribution D2.

For example, the feature amount extraction unit 300b extracts the median value S1_{Me} from the first distribution D1 and extracts the median value S2_{Me} from the second distribution D2 (see Fig. 5).

For example, the feature amount extraction unit 300b extracts the mode value S1_{Mo} from the first distribution D1 and extracts the mode value S2_{Mo} from the second distribution D2 (see Fig. 6).

For example, the feature amount extraction unit 300b extracts a proportion (for example, 0.5) of numbers of signals (for example, 400 and 200 in Fig. 3) whose signal values respectively match a plurality of values (for example, 15 and 45 in Fig. 3) from the first distribution D1, and extracts a proportion (for example, 0.78) of the numbers of signals (for example, 250 and 320 in Fig. 3) whose signal values respectively match the plurality of values (for example, 15 and 45 in Fig. 3) from the second distribution D2.

In the next step S16, the processing system 300-2 compares the feature amounts of the first and second distributions D1 and D2. Specifically, the comparison unit 300e compares the corresponding feature amounts of the input first and second distributions D1 and D2, and outputs the comparison result (for example, a difference, a ratio, or the like between the feature amounts) to the determination unit 300c.

For example, the comparison unit 300e obtains a difference between the input average value S1_{Ave} and average value S2_{Ave}, and outputs the difference to the determination unit 300c.

For example, the comparison unit 300e obtains a difference between the number of signals whose signal values are equal to or less than the average value S1_{Ave} and the number of signals whose signal values are equal to or less than the average value S2_{Ave}, and outputs the difference to the determination unit 300c.

For example, the comparison unit 300e obtains a difference between the input median value S1_{Me} and the median value S2_{Me}, and outputs the difference to the determination unit 300c.

For example, the comparison unit 300e obtains a difference between the input mode value S1_{Mo} and the mode value S2_{Mo}, and outputs the difference to the determination unit 300c.

Note that the determination unit 300c may output a result of the determination as described above (for example, a mental condition of the living body LB according to the magnitude of the difference) in evaluation stages. For example, determination results may be output in ten stages in which a case where the living body LB is in the most relaxed state is set to 1 and a case where the living body LB is in the tense state is set to 10.

In the next step S17, the processing system 300-2 determines a condition of the living body LB. Specifically, the determination unit 300c determines the condition of the living body LB according to the input comparison result (for example, difference, ratio, or the like between the feature amounts).

For example, the determination unit 300c determines that the living body LB is in a tenser state as the difference between the input feature amounts is larger, and determines that the living body LB is in a more relaxed state as the difference is smaller.

In the final step S18, the processing system 300-2 determines whether or not to end the processing. Specifically, the processing system 300-2 ends the processing, for example, when the biological sensor according to the modified example of Example 1 is powered off. The flow ends if the determination in step S18 is positive, and returns to step S11 if the determination is negative.

Note that the second distribution D2 is stored in the storage unit 300d in advance in the biological sensor according to the modified example of Example 1. However, instead of or in addition to this, the second distribution D2 may be acquired and updated as needed using the biological sensor.

In the biological sensor according to the modified example of Example 1, the determination unit 300c may also have the function of the comparison unit 300e. In this case, the comparison unit 300e is unnecessary.

### 8. <Effect of Biological Sensor According to Modified Example of Example 1 of Embodiment of Present Technology>

In the biological sensor according to the modified example of Example 1, the processing system 300-2 compares the feature amounts of the first and second distributions D1D and D2 to determine the condition of the living body LB (for example, the mental condition according to a sweating state of the living body LB). Therefore, the condition of the living body LB can be determined more accurately.

The feature amount of the second distribution D2 is the feature amount (of the same type) corresponding to the feature amount of the first distribution D1.

For example, the feature amount of the first distribution D1 may be a proportion of numbers of signals whose signal values respectively match a plurality of values among signals of the respective sites in the first distribution D1, and the feature amount of the second distribution D2 may be a proportion of numbers of signals whose signal values respectively match the plurality of values among signals of the respective sites in the second distribution D2.

### 9. <Biological Sensors According to Examples 2 to 20 of Embodiment of Present Technology>

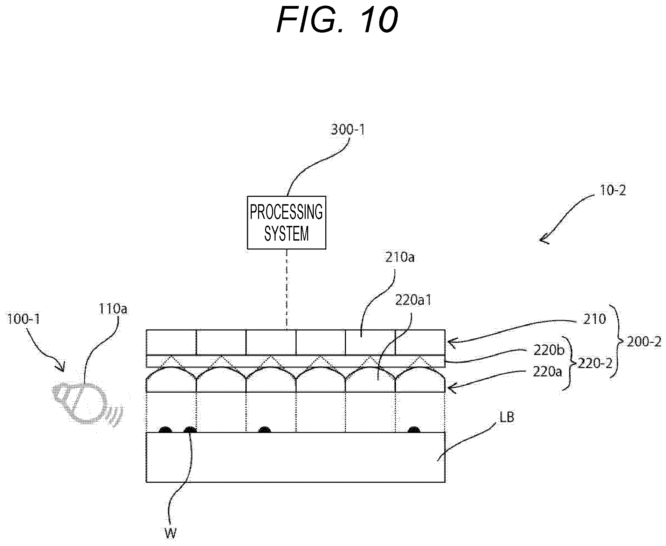

Hereinafter, biological sensors 10-2 to 10 to 20 according to Example 2 to 20 of the embodiment will be described with reference to Figs. 10 to 28.

### (Biological Sensor According to Example 2)

As illustrated in Fig. 10, a biological sensor 10-2 according to Example 2 has a configuration similar to that of that of the biological sensor 10-1 according to Example 1 except that a positional relationship between the lens array 220a and the wavelength selection filter 220b in an optical system of a light receiving system is reversed from that of the biological sensor 10-1 according to Example 1.

In the biological sensor 10-2, a wavelength selection filter 220b of an optical system 220-2 is arranged between the lens array 220a of the optical system 220-2 of a light receiving system 200-2 and the light receiving element array 210.

That is, in the optical system 220-2, the lens array 220a and the wavelength selection filter 220b are arranged adjacent to each other such that the lens array 220a is arranged on the living body LB side and the wavelength selection filter 220b is arranged on the light receiving element array 210 side.

As described above, the optical system 220-2 includes the light receiving lens 220a1 that guides light having a wavelength within the wavelength band WB to the corresponding light receiving element 210a via the wavelength selection filter 220b in the biological sensor 10-2 according to Example 2.

The biological sensor 10-2 according to Example 2 described above can also obtain an effect similar to that of that of the biological sensor 10-1 according to Example 1.

### (Biological Sensor According to Example 3)

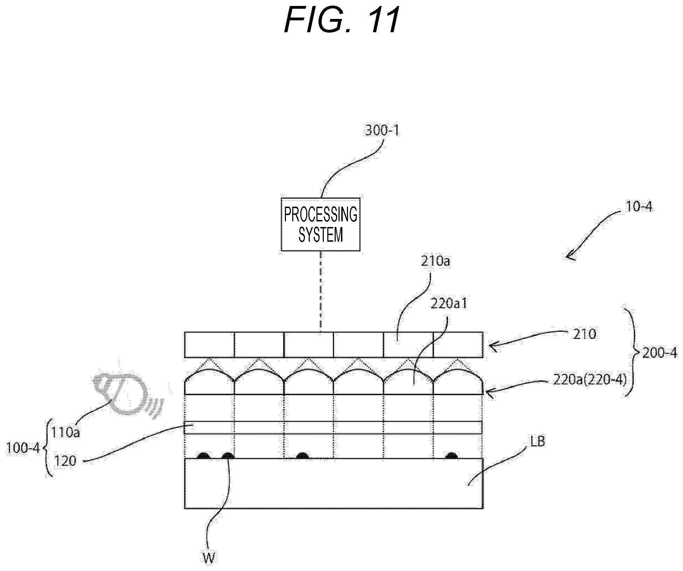

As illustrated in Fig. 11, a biological sensor 10-4 according to Example 3 has a configuration similar to that of the biological sensor 10-1 according to Example 1 except that an irradiation system includes a wavelength selection filter instead of a light receiving system.

An irradiation system 100-4 of the biological sensor 10-4 is provided with a wavelength selection filter 120 arranged on an optical path between the light source 110a and the living body LB. The wavelength selection filter 120 is substantially the same as the wavelength selection filter 220b of the biological sensor 10-1 according to Example 1.

That is, the irradiation system 100-4 includes: the light source 110a that emits light having a wavelength within the wavelength band WB and light having a wavelength outside the wavelength band WB; and the wavelength selection filter 120 that selectively passes the light within the wavelength band WB out of the light within the wavelength band WB and the light outside the wavelength band WB emitted from the light source 110a.

An optical system 220-4 of a light receiving system 200-4 of the biological sensor 10-4 includes the lens array 220a including the plurality of light receiving lenses 220a1 that guides the light having the wavelength within the wavelength band WB that has passed through the wavelength selection filter 120 and reflected from a corresponding site of the living body LB to the corresponding light receiving element 210a.

In the biological sensor 10-4 configured as described above, out of the light having the wavelength within the wavelength band WB and the light having the wavelength outside the wavelength band WB emitted from the light source 110a, the light having the wavelength within the wavelength band WB passes through the wavelength selection filter 120 and is emitted to the living body LB as irradiation light. The light having the wavelength within the wavelength band WB emitted to the living body LB and reflected from each of a plurality of sites of the living body LB passes through the wavelength selection filter 120 again, and is guided to the corresponding light receiving element 210a via the corresponding light receiving lens 220a1.

The biological sensor 10-4 according to Example 3 described above also exhibits an effect similar to that of the biological sensor 10-1 according to Example 1.

### (Biological Sensor According to Example 4)

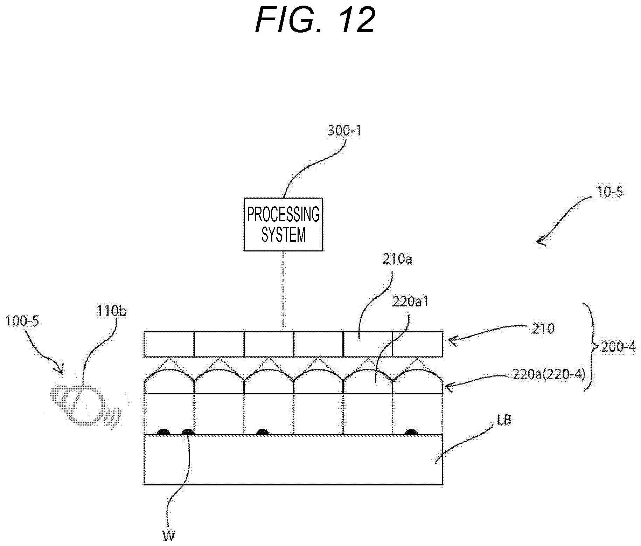

As illustrated in Fig. 12, a biological sensor 10-5 according to Example 4 has a configuration similar to that of the biological sensor 10-1 according to Example 1 except that an irradiation system 100-5 includes a light source 110b that emits only light within the wavelength band WB and the light receiving system 200-4 does not include a wavelength selection filter.

That is, in the biological sensor 10-5, irradiation light includes only light having a wavelength within the wavelength band WB, and the optical system 220-4 of the light receiving system 200-4 includes the lens array 220a including the light receiving lens 220a1 that guides the light having the wavelength within the wavelength band WB, emitted from the irradiation system 100-5 and reflected from each of a plurality of sites of the living body LB, to the light receiving element 210a corresponding to the site.

In the biological sensor 10-5 configured as described above, the living body LB is irradiated with light having the wavelength within the wavelength band WB emitted from the light source 110b as the irradiation light. The light having the wavelength within the wavelength band WB emitted to the living body LB and reflected from each of a plurality of sites of the living body LB is guided to the corresponding light receiving element 210a via the light receiving lens 220a1 corresponding to the site.

The biological sensor 10-5 according to Example 4 described above also exhibits an effect similar to that of the biological sensor 10-1 according to Example 1, and can be downsized since no wavelength selection filter is provided.

### (Biological Sensor According to Example 5)

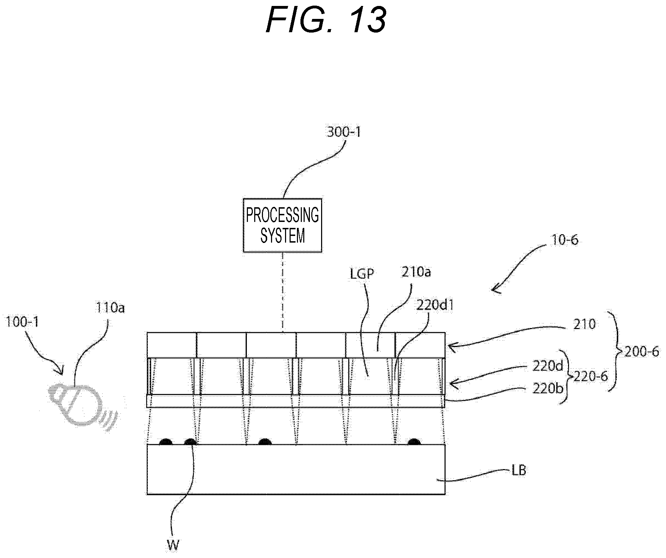

As illustrated in Fig. 13, a biological sensor 10-6 according to Example 5 has a configuration similar to that of the biological sensor 10-1 according to Example 1 except that an optical system 220-6 of a light receiving system 200-6 includes a light shielding member 220d (optical member) instead of a lens array.

The light shielding member 220d is arranged between the wavelength selection filter 220b and the light receiving element array 210.

The light shielding member 220d has light shielding walls 220d1 forming light guide paths LGP respectively corresponding to the plurality of light receiving elements 210a. The light shielding walls 220d1 are provided so as to surround each of the light guide paths LGP. The adjacent light guide paths LGP are separated by the light shielding wall 220d1.

In the biological sensor 10-6 configured as described above, light having a wavelength within the wavelength band WB out of light emitted to the living body LB and reflected from each of a plurality of sites of the living body LB passes through the wavelength selection filter 220b and enters the corresponding light receiving element 210a via the corresponding light guide path LGP.

According to the biological sensor 10-6 described above, it is possible to obtain substantially an effect similar to that of that of the biological sensor 10-1 of Example 1.

### (Biological Sensor According to Example 6)

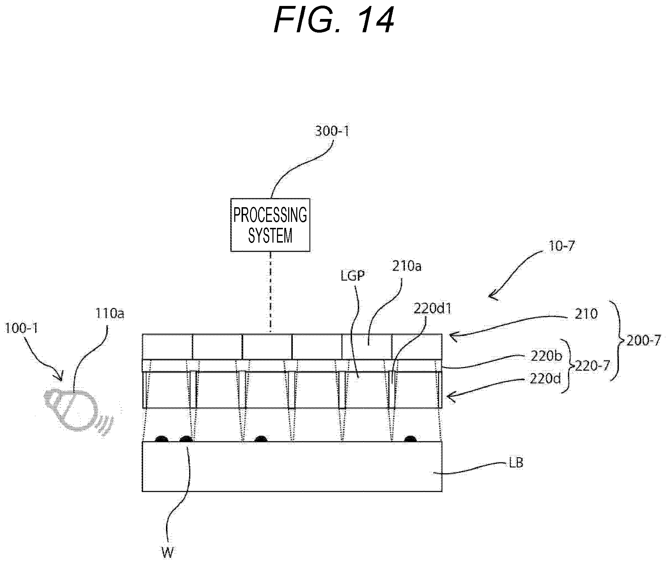

As illustrated in Fig. 14, a biological sensor 10-7 according to Example 6 has a configuration similar to that of the biological sensor 10-6 according to Example 5 except that a positional relationship between the wavelength selection filter 220b and the light shielding member 220d is different in an optical system of a light receiving system.

In the biological sensor 10-7, the light shielding member 220d is arranged on the living body LB side in an optical system 220-7 of a light receiving system 200-7, and the wavelength selection filter 220b is arranged on the lens array 220a side.

In the biological sensor 10-7 configured as described above, light emitted from the irradiation system 100-1 and reflected from each of a plurality of sites of the living body LB is guided by the corresponding light guide path LGP of the light shielding member 220d, and light having a wavelength within the wavelength band WB out of the guided light passes through the wavelength selection filter 220b and enters the corresponding light receiving element 210a.

According to the biological sensor 10-7 described above, an effect similar to that of that of the biological sensor 10-6 of Example 5 can be obtained.

### (Biological Sensor According to Example 7)

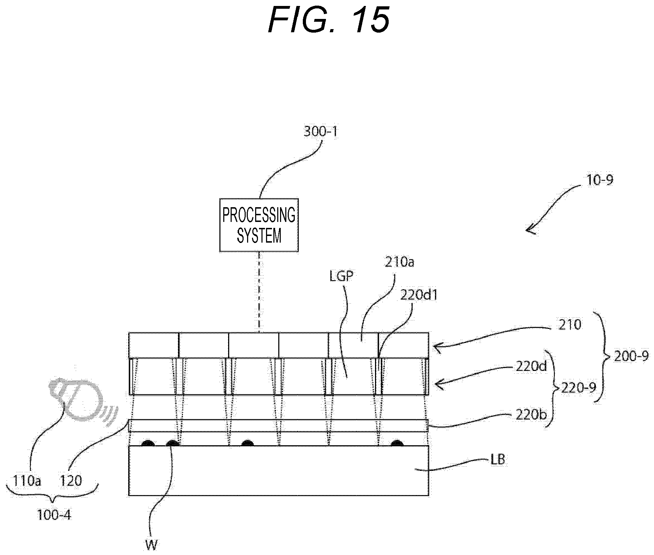

As illustrated in Fig. 15, the biological sensor 10-9 according to Example 7 has a configuration similar to that of the biological sensor 10-1 according to Example 1 except that an irradiation system includes a wavelength selection filter instead of a light receiving system.

The irradiation system 100-4 of the biological sensor 10-9 includes the wavelength selection filter 120 arranged on an optical path between the light source 110a and the living body LB. The wavelength selection filter 120 is substantially the same as the wavelength selection filter 220b of the biological sensor 10-1 according to Example 1.

That is, the irradiation system 100-4 includes: the light source 110a that emits light having a wavelength within the wavelength band WB and light having a wavelength outside the wavelength band WB; and the wavelength selection filter 120 that selectively passes the light within the wavelength band WB out of the light within the wavelength band WB and the light outside the wavelength band WB emitted from the light source 110a.

An optical system 220-9 of a light receiving system 200-9 of the biological sensor 10-9 includes the light shielding member 220d that guides the light having the wavelength within the wavelength band WB that has passed through the wavelength selection filter 120 and reflected from each of a plurality of sites of the living body LB to the light receiving element 210a corresponding to the site.

In the biological sensor 10-9 configured as described above, out of the light having the wavelength within the wavelength band WB and the light having the wavelength outside the wavelength band WB emitted from the light source 110a, the light having the wavelength within the wavelength band WB passes through the wavelength selection filter 120 and is emitted to the living body LB as irradiation light. The light having the wavelength within the wavelength band WB emitted to the living body LB and reflected from each of a plurality of sites of the living body LB passes through the wavelength selection filter 120 again, and is guided to the corresponding light receiving element 210a via the corresponding light guide path LGP of the light shielding member 220d.

The biological sensor 10-9 according to Example 7 described above also exhibits an effect similar to that of the biological sensor 10-6 according to Example 5.

### (Biological Sensor According to Example 8)

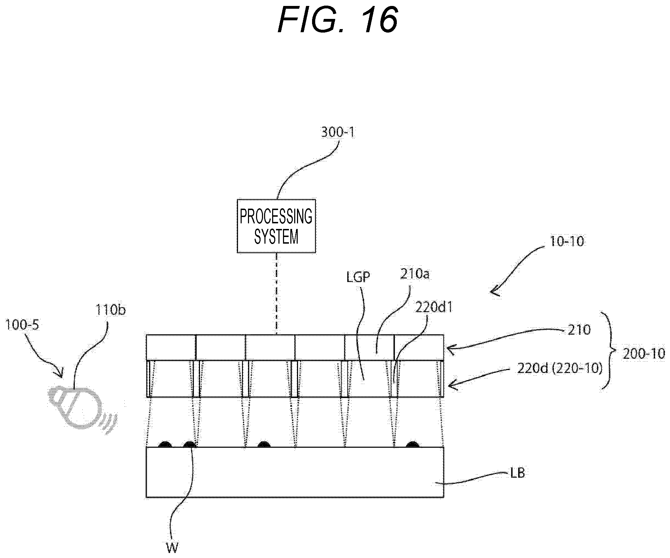

As illustrated in Fig. 16, a biological sensor 10-10 according to Example 8 has a configuration similar to that of the biological sensor 10-6 according to Example 5 except that the irradiation system 100-5 includes the light source 110b that emits only light within the wavelength band WB and a light receiving system 200-10 does not include a wavelength selection filter.

That is, in the biological sensor 10-10, irradiation light includes only light having a wavelength within the wavelength band WB, and an optical system 220-10 of the light receiving system 200-10 includes the light shielding member 220d that guides the light having the wavelength within the wavelength band WB, emitted from the irradiation system 100-5 and reflected from each of a plurality of sites of the living body LB, to the light receiving element 210a corresponding to the site.

In the biological sensor 10-10 configured as described above, the living body LB is irradiated with light having the wavelength within the wavelength band WB emitted from the light source 110b as the irradiation light. The light having the wavelength within the wavelength band WB emitted to the living body LB and reflected from each of the plurality of sites of the living body LB is guided to the corresponding light receiving element 210a via the corresponding light guide path LGP of the light shielding member 220d.

The biological sensor 10-10 according to Example 8 described above also exhibits an effect similar to that of the biological sensor 10-6 according to Example 5, and can be downsized since no wavelength selection filter is provided.

### (Biological Sensor According to Example 9)

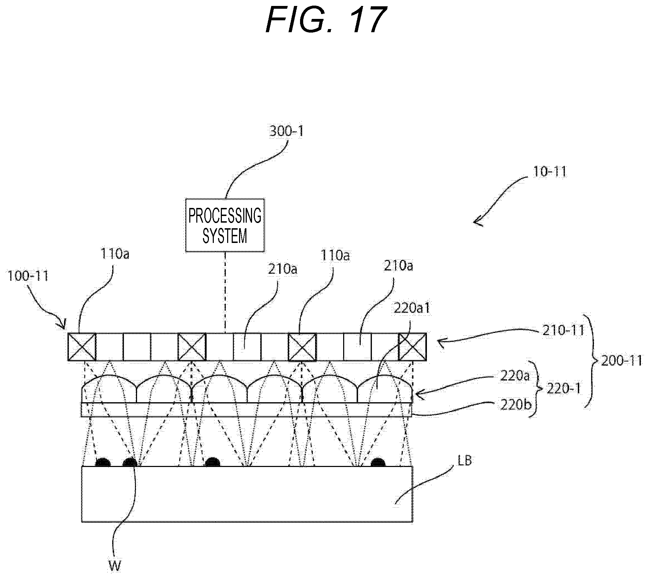

As illustrated in Fig. 17, a biological sensor 10-11 according to Example 9 has the similar configuration as the biological sensor 10-1 according to Example 1 except that an irradiation system 100-11 has a light source array including a plurality of the light sources 110a, and the light source array and a light receiving element array 210-11 including the plurality of light receiving elements 210a are integrally provided along the same plane.

The irradiation system 100-11 of the biological sensor 10-11 includes one or more (for example, a plurality of) light sources 110a which are arranged between two adjacent light receiving elements 210a among the plurality of light receiving elements 210a arranged in an array and emit light having a wavelength within the wavelength band WB and light having a wavelength outside the wavelength band WB.

Conversely, a light receiving system 200-11 of the biological sensor 10-11 includes one or more (for example, a plurality of) light receiving elements 210a arranged between two adjacent light sources 110a among the plurality of light sources 110a arranged in the array.

In the example of Fig. 17, the plurality of light sources 110a of the light source array and the plurality of light receiving elements 210a of the light receiving element array 210-11 are arranged in an array along the same plane as a whole. Here, as an example, three light receiving elements 210a are arranged between two adjacent light sources 110a.

Each of the light sources 110a is arranged at a position corresponding to a boundary between two adjacent light receiving lenses 220a1 of the lens array 220a of the optical system 220-1 in the in-plane direction of the light source array.

In a generation sensor 10-11, the light having the wavelength within the wavelength band WB and the light having the wavelength outside the wavelength band WB emitted from each of the light sources 110a are incident across the two adjacent light receiving lenses 220a1 and diffused by the two light receiving lenses 220a1. Out of the diffused light, light having the wavelength within the wavelength band WB passes through the wavelength selection filter 220b and is emitted across two adjacent sites of the living body LB. The light having the wavelength within the wavelength band WB reflected from each of the two adjacent sites of the living body LB passes through the wavelength selection filter 220b again, and is collected on the corresponding light receiving element 210a by the corresponding light receiving lens 220a1.

According to the biological sensor 10-11, an effect similar to that of the biological sensor 10-1 according to Example 1 is exhibited, and downsizing can be achieved since the plurality of light sources 110a and the plurality of light receiving elements 210a are integrally arranged in the array.

### (Biological Sensor According to Example 10)

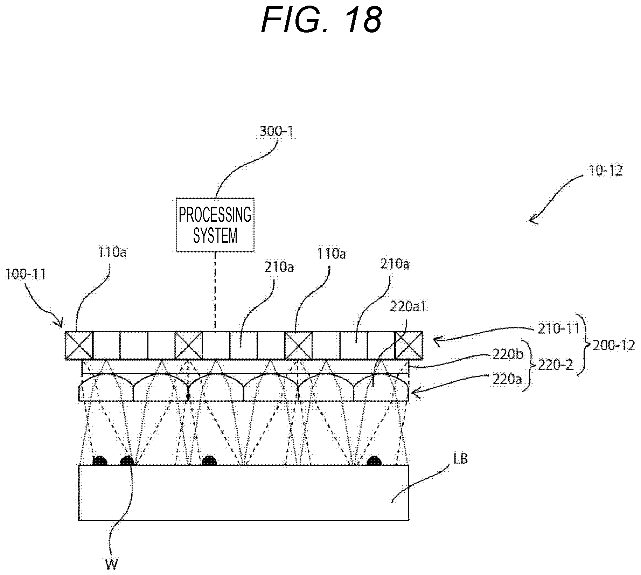

As illustrated in Fig. 18, a biological sensor 10-12 according to Example 10 has a configuration similar to that of the biological sensor 10-11 according to Example 9 except that a positional relationship between a lens array and a wavelength selection filter in an optical system of a light receiving system is different.

In the biological sensor 10-12, the wavelength selection filter 220b of the optical system 220-2 is arranged between the lens array 220a of the optical system 220-2 of a light receiving system 200-12 and the light receiving element array 210-11.

That is, in the optical system 220-2, the lens array 220a and the wavelength selection filter 220b are arranged adjacent to each other such that the lens array 220a is arranged on the living body LB side and the wavelength selection filter 220b is arranged on the light receiving element array 210 side.

As described above, the optical system 220-2 includes the light receiving lens 220a1 that guides light having a wavelength within the wavelength band WB to the corresponding light receiving element 210a via the wavelength selection filter 220b in the biological sensor 10-12 according to Example 10.

The biological sensor 10-12 according to Example 10 described above can obtain an effect similar to that of that of the biological sensor 10-11 according to Example 9.

### (Biological Sensor According to Example 11)

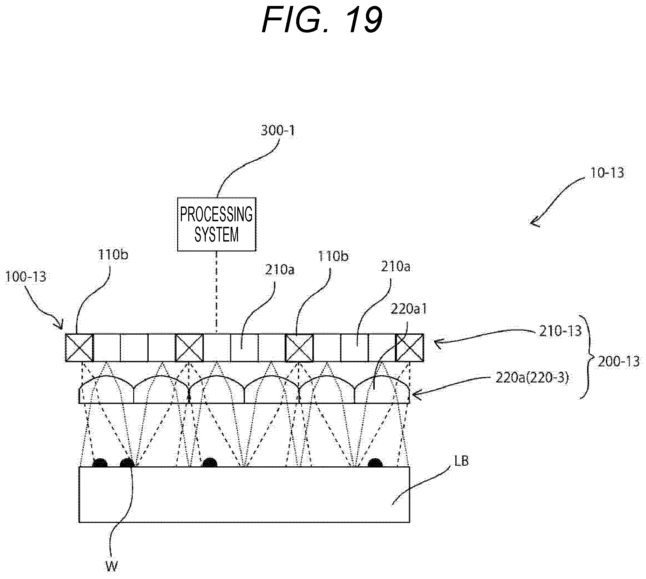

As illustrated in Fig. 19, a biological sensor 10-13 according to Example 11 has a configuration similar to that of the biological sensor 10-11 according to Example 9 except that an irradiation system 100-13 includes the light sources 110b that emit only light within the wavelength band WB and a light receiving system 200-13 does not include a wavelength selection filter.

That is, in the biological sensor 10-13, irradiation light includes only light having a wavelength within the wavelength band WB, and an optical system 220-3 of the light receiving system 200-13 guides the light having the wavelength within the wavelength band WB, emitted from the irradiation system 100-13 and reflected from each of a plurality of sites of the living body LB, to the light receiving element 210a corresponding to the site, and includes the lens array 220a including a plurality of the light receiving lenses 220a1.

In the biological sensor 10-13 configured as described above, the light having the wavelength within the wavelength band WB emitted from each of the light sources 110b is emitted across two adjacent sites of the living body LB as the irradiation light. The light having the wavelength within the wavelength band WB emitted to the two adjacent sites of the living body LB and reflected from each of the two sites is collected on the corresponding light receiving element 210a by the light receiving lens 220a1 corresponding to each of the sites.

The biological sensor 10-13 according to Example 11 described above also exhibits an effect similar to that of the biological sensor 10-11 according to Example 9, and can be downsized since no wavelength selection filter is provided.

### (Biological Sensor According to Example 12)

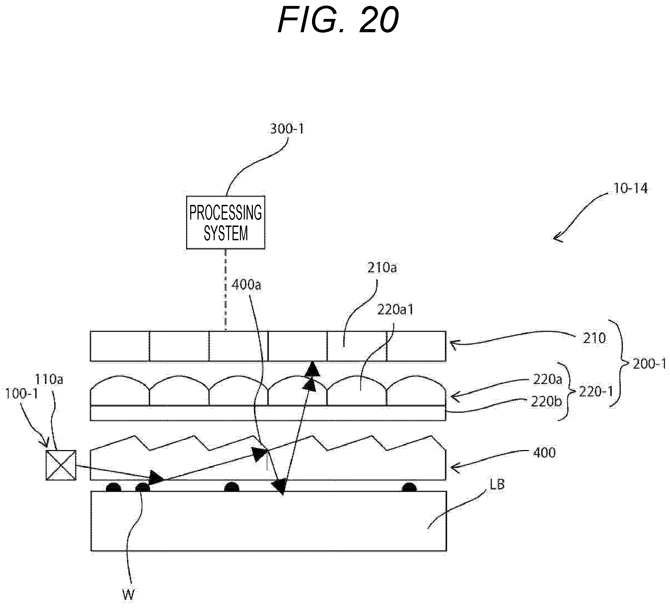

As illustrated in Fig. 20, a biological sensor 10-14 according to Example 12 has a configuration similar to that of the biological sensor 10-1 according to Example 1 except that a light guide plate 400 arranged between the optical system 220-1 and the living body LB is provided.

In the biological sensor 10-14, the irradiation system 100-1 includes a light source 100a that emits light having a wavelength within the wavelength band WB and light having a wavelength outside the wavelength band WB.

The light source 110a is arranged on a side of one end surface of the light guide plate 400 in a state where an emission direction is directed to the one end surface. More specifically, the emission direction of the light source 110a is set such that light emitted from the light source 110a, that is, the light being incident on the light guide plate 400 from the one end surface of the light guide plate 400, is totally reflected by a surface of the light guide plate 400 on the living body LB side.

The light guide plate 400 guides the light having the wavelength within the wavelength band WB and the light having the wavelength outside the wavelength band WB out of the light emitted from the light source 100a, and causes the light having the wavelength within the wavelength band WB and the light outside the wavelength band WB to be incident on each of a plurality of sites of the living body LB.

More specifically, the light guide plate 400 includes, on the optical system 220-1 side, a plurality of diffraction units 400a respectively corresponding to the plurality of light receiving elements 210a. The light guide plate 400 is transparent with respect to at least the light having the wavelength within the wavelength band WB. Each of the plurality of diffraction units 400a reflects and diffracts the incident light toward a corresponding site of the living body LB.

In the biological sensor 10-14 configured as described above, most of the light emitted from the light source 110a enters the light guide plate 400 from the one end surface of the light guide plate 400, and is totally reflected at total reflection angles, different from each other, on the surface of the light guide plate 400 on the living body LB side. Each beam of the light totally reflected at the total reflection angles, different from each other, is reflected and diffracted by the corresponding diffraction unit 400a, is transmitted through the surface of the light guide plate 400 on the living body LB side, and enters a corresponding site of the living body LB. The light incident on the site is reflected by the site and is transmitted through the corresponding diffraction unit 400a of the light guide plate 400. Out of the transmitted light, only the light having the wavelength within the wavelength band WB passes through the wavelength selection filter 220b and is collected on the corresponding light receiving element 210a by the corresponding light receiving lens 220a1.

The biological sensor 10-14 according to Example 12 described above also exhibits an effect similar to that of the biological sensor 10-1 according to first Example 1 described above, and can more accurately guide the reflected light from each of the sites of the living body LB to the corresponding light receiving element 210a by the light guide plate 400.

Note that the light guide plate 400 may be transparent only to the light having the wavelength within the wavelength band WB out of the light emitted from the light source 110a. In this case, the light having the wavelength outside the wavelength band WB can be shielded, and thus, the wavelength selection filter 220b is unnecessary.

### (Biological Sensor According to Example 13)

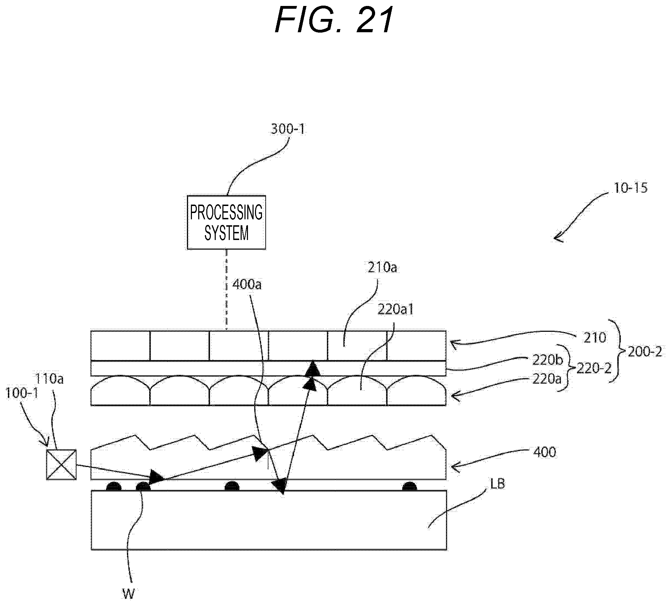

As illustrated in Fig. 21, a biological sensor 10-15 according to Example 13 has a configuration similar to that of the biological sensor 10-14 according to Example 12 except that a positional relationship between the lens array 220a and the wavelength selection filter 220b in an optical system of a light receiving system is different.

In the biological sensor 10-15, the wavelength selection filter 220b of the optical system 220-2 is arranged between the lens array 220a of the optical system 220-2 of the light receiving system 200-2 and the light receiving element array 210.

That is, in the optical system 220-2, the lens array 220a and the wavelength selection filter 220b are arranged adjacent to each other such that the lens array 220a is arranged on the living body LB side and the wavelength selection filter 220b is arranged on the light receiving element array 210 side.

As described above, the optical system 220-2 includes the light receiving lens 220a1 that guides light having a wavelength within the wavelength band WB to the corresponding light receiving element 210a via the wavelength selection filter 220b in the biological sensor 10-15 according to Example 13.

The biological sensor 10-15 according to Example 13 described above exhibits an effect similar to that of the biological sensor 10-14 according to Example 12.

### (Biological Sensor According to Example 14)

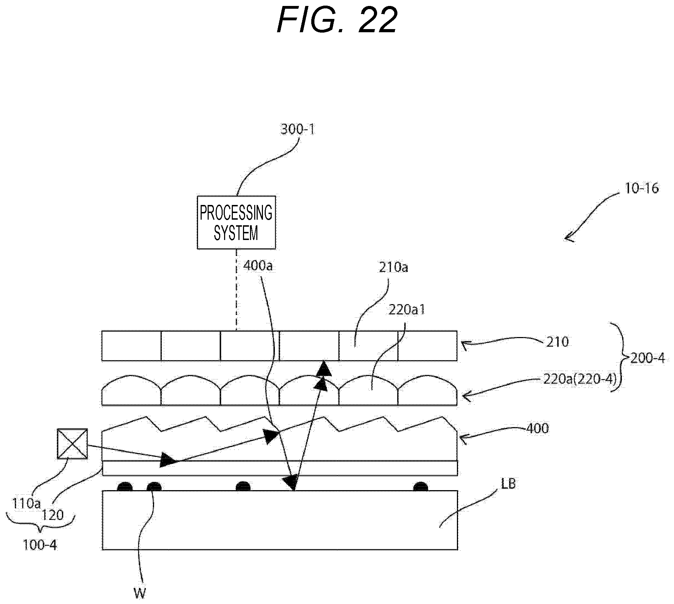

As illustrated in Fig. 22, a biological sensor 10-16 according to Example 14 has a configuration similar to that of the biological sensor 10-14 according to Example 12 except that an irradiation system includes a wavelength selection filter instead of a light receiving system.

The irradiation system 100-4 of the biological sensor 10-16 includes the wavelength selection filter 120 arranged on an optical path between the light source 110a and the living body LB. The wavelength selection filter 120 is substantially the same as the wavelength selection filter 220b of the biological sensor 10-1 according to Example 1.

The light source 110a emits light having a wavelength within the wavelength band WB and light having a wavelength outside the wavelength band WB.

The wavelength selection filter 120 selectively passes the light within the wavelength band WB out of the light within the wavelength band WB and the light outside the wavelength band WB emitted from the light source 110a.

The optical system 220-4 of the light receiving system 200-4 of the biological sensor 10-16 includes the light receiving lens 220a1 that guides the light having the wavelength within the wavelength band WB that has passed through the wavelength selection filter 120 and reflected from each of a plurality of sites of the living body LB to the light receiving element 210a corresponding to the site.

In the biological sensor 10-16 configured as described above, each beam of the light emitted from the light source 110a and totally reflected by a surface of the light guide plate 400 on the living body LB side is diffracted by the corresponding diffraction unit 400a, transmitted through the surface of the light guide plate 400 on the living body LB side, and incident on the wavelength selection filter 120. Each beam of the light having the wavelength within the wavelength band WB that has passed through the wavelength selection filter 120 is reflected by a corresponding site of the living body LB, enters the corresponding light receiving lens 220a1 via the wavelength selection filter 120 and the corresponding diffraction unit 400a, and is collected on the corresponding light receiving element 210a by the light receiving lens 220a1.

The biological sensor 10-16 according to Example 14 described above also exhibits an effect similar to that of the biological sensor 10-14 according to Example 12.

### (Biological Sensor According to Example 15)

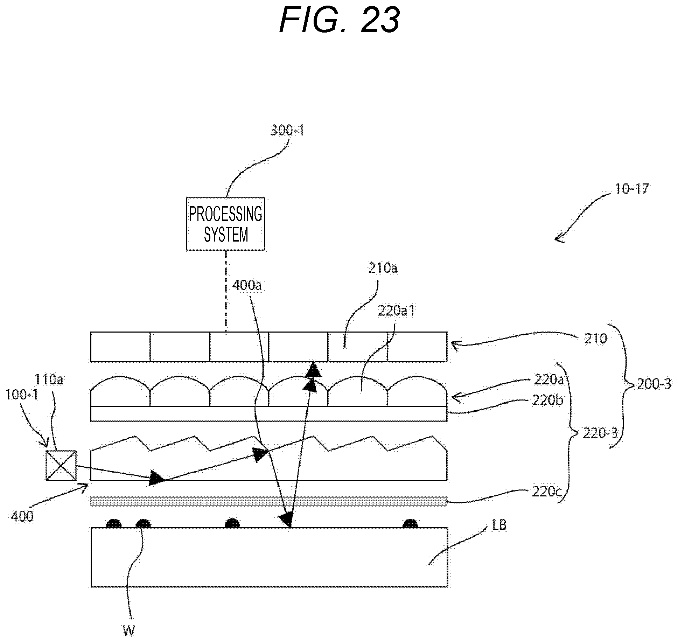

As illustrated in Fig. 23, a biological sensor 10-17 according to Example 15 has a configuration similar to that of the biological sensor 10-14 according to Example 12 except that a circularly polarizing plate 220c is arranged between the light receiving element array 210 and the living body LB.

According to the biological sensor 10-17, sweat and surface reflection at the surface (skin) of the living body LB can be suppressed by the circularly polarizing plate 220c, and thus, unnecessary noise can be reduced, and stable measurement can be performed.

Here, as an example, the optical system 220-3 of a light receiving system 200-3 includes the circularly polarizing plate 220c arranged between the light guide plate 400 and the living body LB in the biological sensor 10-17.

### (Biological Sensor According to Example 16)

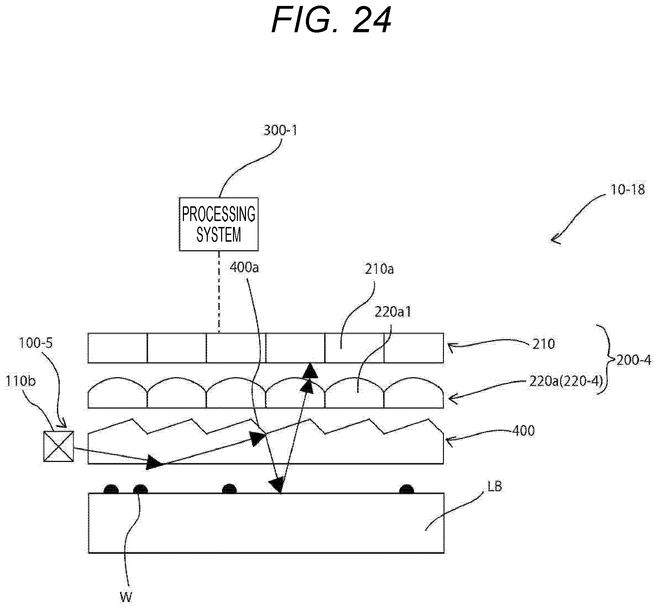

As illustrated in Fig. 24, a biological sensor 10-18 according to Example 16 has a configuration similar to that of the biological sensor 10-14 according to Example 12 except that the irradiation system 100-5 includes the light source 110b that emits only light within the wavelength band WB and the light receiving system 200-4 does not include a wavelength selection filter.

That is, in the biological sensor 10-18, irradiation light includes only light having a wavelength within the wavelength band WB, and the optical system 220-4 of the light receiving system 200-4 includes the lens array 220a including the light receiving lens 220a1 that guides the light of the wavelength within the wavelength band WB, which has been emitted from the irradiation system 100-5 via the light guide plate 400, reflected from each of a plurality of sites of the living body LB, and transmitted through the light guide plate 400, to the light receiving element 210a corresponding to the site.

In the biological sensor 10-18 configured as described above, most of the light having the wavelength within the wavelength band WB emitted from each of the light sources 110b enters the light guide plate 400 from one end surface of the light guide plate 400, and is totally reflected at total reflection angles, different from each other, on the surface of the light guide plate 400 on the living body LB side. Each beam of the light totally reflected at the total reflection angles, different from each other, is reflected and diffracted by the corresponding diffraction unit 400a, is transmitted through the surface of the light guide plate 400 on the living body LB side, and enters a corresponding site of the living body LB. The light incident on the site is reflected by the site, is incident on the light guide plate 400 again, and is transmitted through the corresponding diffraction unit 400a of the light guide plate 400. The transmitted light is collected on the corresponding light receiving element 210a by the corresponding light receiving lens 220a1.

The biological sensor 10-18 according to Example 16 described above also exhibits an effect similar to that of the biological sensor 10-14 according to Example 12, and can be downsized since no wavelength selection filter is provided.

### (Biological Sensor According to Example 17)

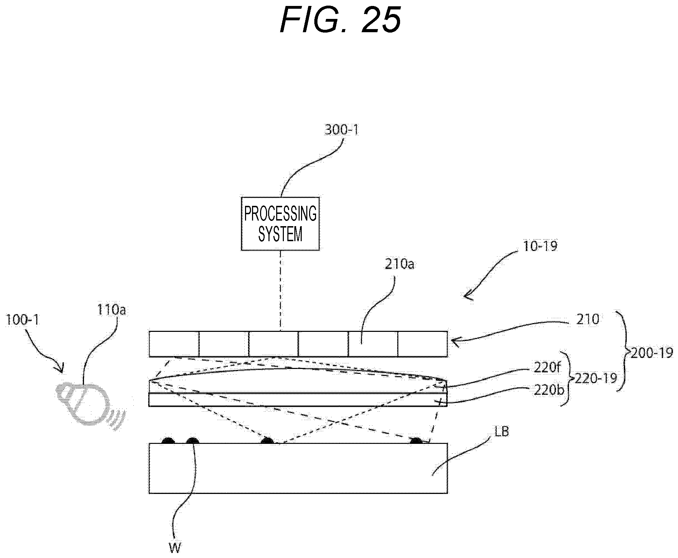

As illustrated in Fig. 25, a biological sensor 10-19 according to Example 17 has a configuration similar to that of the biological sensor 10-1 according to Example 1 except that a light receiving system includes a lens instead of a lens array.

In the biological sensor 10-19, an optical system 220-19 of a light receiving system 200-19 includes a lens 220f (optical member) corresponding to the light receiving element array 210 including the plurality of light receiving elements 210a. The lens 220f is, for example, a lens that is convex toward the light receiving element array 210.

In the biological sensor 10-19, only light having a wavelength within the wavelength band WB out of light emitted from the light source 110a and reflected from each of a plurality of sites of the living body LB passes through the wavelength selection filter 220b and is incident on the lens 220f. The light having the wavelength within the wavelength band WB incident on the lens 220f is collected on the corresponding light receiving element 210a by the lens 220f.

According to the biological sensor 10-19 described above, an effect similar to that of that of an optical sensor 10-1 according to Example 1 is achieved, and positioning between the optical system 220-19 and the light receiving element array 210 is easy since the optical system 220-19 includes the single lens 220f, a design is established similarly to a camera, and the lens 220f can be obtained at low cost.

### (Biological Sensor According to Example 18)

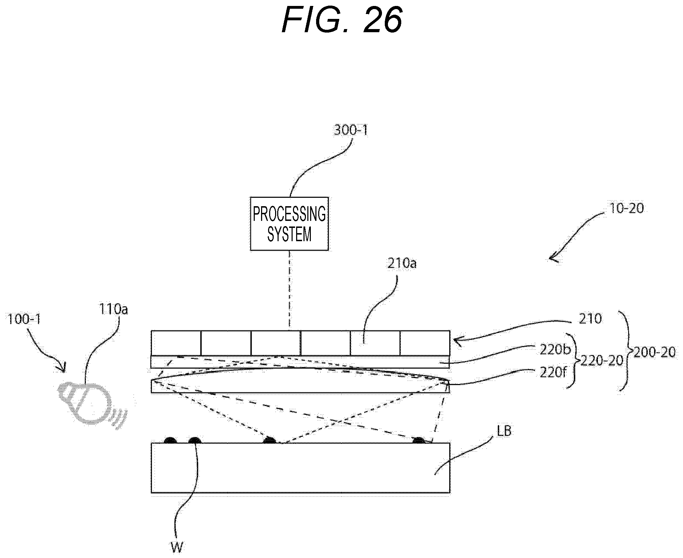

As illustrated in Fig. 26, a biological sensor 10-20 according to Example 18 has a configuration similar to that of the biological sensor 10-19 according to Example 17 except that a positional relationship between a lens array and a wavelength selection filter in an optical system of a light receiving system is different.

In the biological sensor 10-20, the wavelength selection filter 220b of an optical system 220-20 is arranged between the lens 220f of the optical system 220-20 of the light receiving system 200-2 and the light receiving element array 210.

That is, in the optical system 220-20, the lens 220f and the wavelength selection filter 220b are arranged adjacent to each other such that the lens 220f is arranged on the living body LB side and the wavelength selection filter 220b is arranged on the light receiving element array 210 side.

As described above, the optical system 220-20 includes the lens 220f that guides light having a wavelength within the wavelength band WB to the corresponding light receiving element 210a via the wavelength selection filter 220b in the biological sensor 10-20 according to Example 18. In this case as well, an effect similar to that of the biological sensor 10-19 according to Example 17 can be obtained.

### (Biological Sensor According to Example 19)

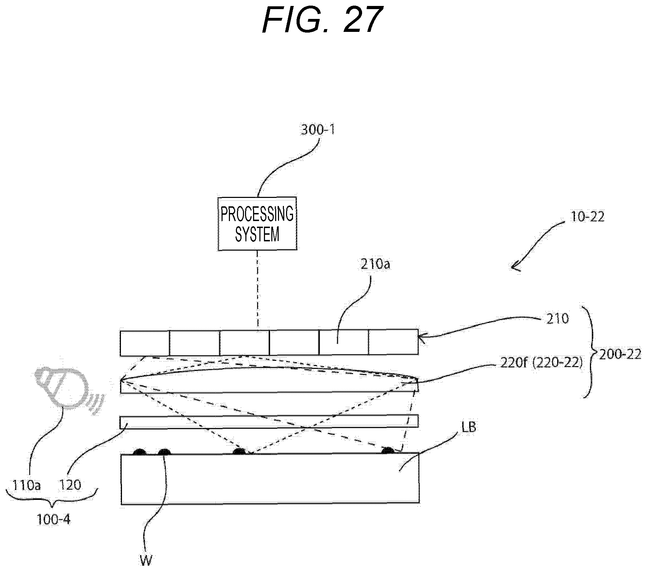

As illustrated in Fig. 27, a biological sensor 10-22 according to Example 19 has a configuration similar to that of the biological sensor 10-19 according to Example 17 except that an irradiation system includes a wavelength selection filter instead of a light receiving system.

The irradiation system 100-4 of the biological sensor 10-22 includes the wavelength selection filter 120 arranged on an optical path between the light source 110a and the living body LB. The wavelength selection filter 120 is substantially the same as the wavelength selection filter 220b of the biological sensor 10-1 according to Example 1.

The light source 110a emits light having a wavelength within the wavelength band WB and light having a wavelength outside the wavelength band WB.

The wavelength selection filter 120 selectively passes the light within the wavelength band WB out of the light within the wavelength band WB and the light outside the wavelength band WB emitted from the light source 110a.

An optical system 220-22 of a light receiving system 200-22 of the biological sensor 10-22 includes the lens 220f that guides the light having the wavelength within the wavelength band WB that has passed through the wavelength selection filter 120 and reflected from each of a plurality of sites of the living body LB to the light receiving element 210a corresponding to the site.

In the biological sensor 10-22 configured as described above, light having a wavelength within the wavelength band WB out of light emitted from the light source 110a is transmitted through the wavelength selection filter 120 and emitted to the living body LB. The light within the wavelength band WB emitted to the living body LB and reflected from each of a plurality of sites of the living body LB is collected on the light receiving element 210a corresponding to the site by the lens 220f.

The biological sensor 10-22 according to Example 19 described above also exhibits an effect similar to that of the biological sensor 10-19 according to Example 17.

### (Biological Sensor According to Example 20)

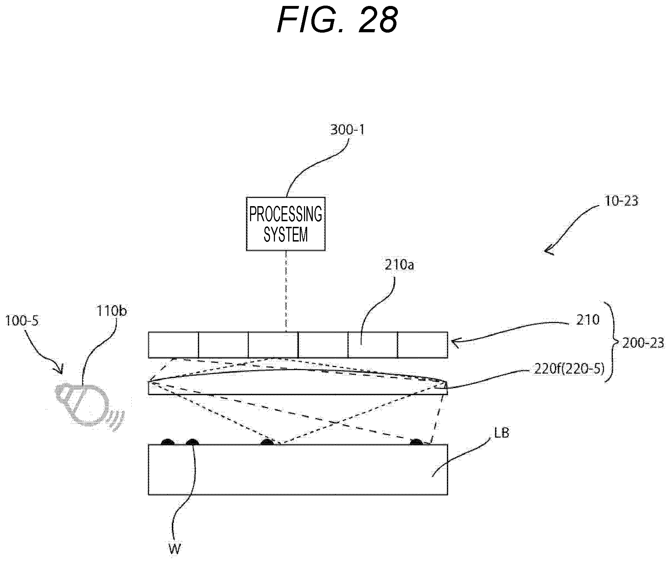

As illustrated in Fig. 28, a biological sensor 10-23 according to Example 20 has a configuration similar to that of the biological sensor 10-19 according to Example 17 except that the irradiation system 100-5 includes the light source 110b that emits only light within the wavelength band WB and a light receiving system 200-23 does not include a wavelength selection filter.

That is, in the biological sensor 10-23, irradiation light includes only light having a wavelength within the wavelength band WB, and the optical system 220-5 of the light receiving system 200-23 includes the lens 220f that guides the light having the wavelength within the wavelength band WB, emitted from the irradiation system 100-5 and reflected from each of a plurality of sites of the living body LB, to the light receiving element 210a corresponding to the site.

In the biological sensor 10-23 configured as described above, the living body LB is irradiated with light having the wavelength within the wavelength band WB emitted from the light source 110b as the irradiation light. The light having the wavelength within the wavelength band WB emitted to the living body LB and reflected from each of a plurality of sites of the living body LB is guided to the corresponding light receiving element 210a by the lens 220f.

The biological sensor 10-23 according to Example 20 described above also exhibits an effect similar to that of the biological sensor 10-19 according to Example 17, and can be downsized since no wavelength selection filter is provided.

### 10. <Modified Example of Present Technology>

The present technology is not limited to the configurations described in the respective examples and modified examples of the above-described embodiment, and can be modified as appropriate.

For example, the configurations of the above-described examples and modified examples may be combined within a range where there is no contradiction from one another.

For example, an optical system of a light receiving system of a biological sensor according to the present technology may include a line sensor in which a plurality of light receiving elements (pixel sensors) is arranged one-dimensionally (linearly). In this case, the line sensor may be combined with a lens array in which a plurality of light receiving lenses is linearly arranged, a light shielding member in which a plurality of light guide paths is linearly arranged, or a single lens.

For example, the biological sensors according to Example 2 to 20 may include the processing system 300-2 instead of the processing system 300-1.

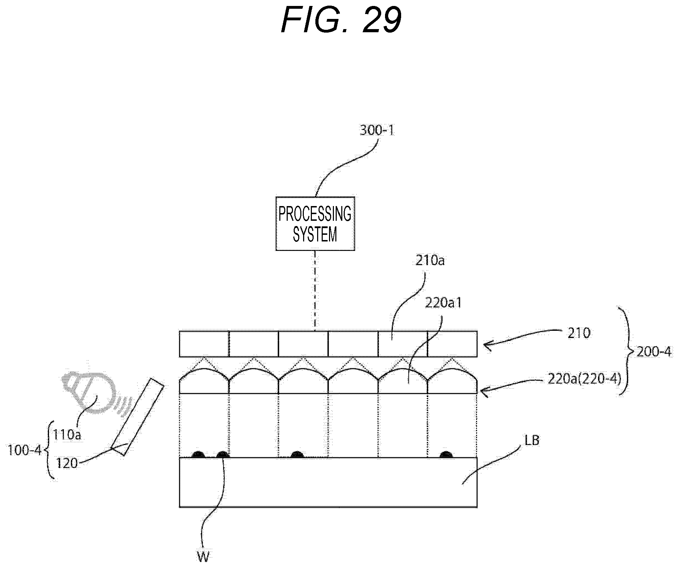

For example, in the biological sensor 10-4 (see Fig. 11) according to Example 3, the wavelength selection filter 120 is arranged between the lens array 220a and the living body LB, but the wavelength selection filter 120 may be arranged between the light source 110a and the living body LB, for example, as in the modified example illustrated in Fig. 29. In the biological sensor 10-8 according to Example 7 (see Fig. 15) and the biological sensor 10-22 according to Example 19 (see Fig. 27) as well, the wavelength selection filter 120 may be arranged similarly to that in Fig. 29. Furthermore, the wavelength selection filter 120 may be arranged between the light source 110a and the light guide plate 400 in the biological sensor 10-12 (see Fig. 22) according to Example 14.

Furthermore, the present technology can also have the following configurations.
(1) A biological sensor including:
   an irradiation system that irradiates a living body with irradiation light including light having a wavelength within a wavelength band including a peak wavelength related to absorption of water;
   a light receiving system that individually receives the light having the wavelength within the wavelength band out of light emitted from the irradiation system and reflected from each of a plurality of sites of the living body; and
   a processing system that determines a condition of the living body on the basis of statistics related to signals of the respective sites output from the light receiving system.
(2) The biological sensor according to (1), in which the processing system determines the condition of the living body by using a first distribution that is a distribution of a number of signals for each of signal values of the signals of the respective sites.
(3) The biological sensor according to (2), in which the processing system determines the condition of the living body from a feature amount of the first distribution.
(4) The biological sensor according to (3), in which the feature amount of the first distribution is a number of signals whose signal values are equal to or less than a reference value of the signals of the respective sites.
(5) The biological sensor according to (4), in which the reference value is an average value of signal values of the signals of the respective sites.
(6) The biological sensor according to (3), in which the feature amount of the first distribution is a median value, an average value, or a mode value of signal values of the signals of the respective sites.
(7) The biological sensor according to any one of (2) to (6), in which the processing system determines the condition of the living body by further using a second distribution that is a distribution of a number of signals for each of signal values of signals of the respective sites according to an inherent reflectance distribution of the plurality of sites.
(8) The biological sensor according to claim 7, in which the processing system compares a feature amount of the first distribution with a feature amount of the second distribution to determine the condition of the living body.
(9) The biological sensor according to (8), in which the feature amount of the second distribution is a feature amount corresponding to the feature amount of the first distribution.
(10) The biological sensor according to (8), in which the feature amount of the first distribution is a proportion of numbers of signals whose signal values respectively match a plurality of values among the signals of the respective sites in the first distribution, and the feature amount of the second distribution is a proportion of numbers of signals whose signal values respectively match the plurality of values among the signals of the respective sites in the second distribution.
(11) The biological sensor according to any one of (1) to (10), in which the light receiving system includes: a light receiving element array including a plurality of light receiving elements respectively corresponding to the plurality of sites; and an optical system corresponding to the plurality of light receiving elements, and the optical system guides the light having the wavelength within the wavelength band out of the light emitted from the irradiation system and reflected from each of the plurality of sites to the light receiving element corresponding to the site.
(12) The biological sensor according to (11), in which the irradiation light includes only the light having the wavelength within the wavelength band, and the optical system includes an optical member that guides the light having the wavelength within the wavelength band, which has been emitted from the irradiation system and reflected by a corresponding site among the sites, to a corresponding light receiving element among the light receiving elements.
(13) The biological sensor according to (11), in which the irradiation light includes the light having the wavelength within the wavelength band and light having a wavelength outside the wavelength band, and the optical system includes a wavelength selection filter that selectively passes the light having the wavelength within the wavelength band out of the light emitted from the irradiation system and reflected from each of the plurality of sites.
(14) The biological sensor according to (13), in which the optical system includes an optical member that guides the light having the wavelength within the wavelength band, which has passed through the wavelength selection filter, to a corresponding light receiving element among the light receiving elements.
(15) The biological sensor according to (13), in which the optical system includes an optical member that guides the light having the wavelength within the wavelength band to a corresponding light receiving element among the light receiving elements via the wavelength selection filter.
(16) The biological sensor according to (11), in which the irradiation system includes: a light source that emits the light having the wavelength within the wavelength band and light having a wavelength outside the wavelength band; and a wavelength selection filter that selectively passes the light within the wavelength band out of the light within the wavelength band and the light outside the wavelength band emitted from the light source.
(17) The biological sensor according to (16), in which the optical system includes an optical member that guides the light having the wavelength within the wavelength band, which has passed through the wavelength selection filter and been reflected by a corresponding site among the sites, to a corresponding light receiving element among the light receiving elements.
(18) The biological sensor according to (11), in which the irradiation system includes at least one light source that is arranged between a pair of the light receiving elements adjacent to each other among the plurality of light receiving elements and emits light including the light having the wavelength within the wavelength band.
(19) The biological sensor according to (11), further including a light guide plate arranged between the optical system and the living body, in which the irradiation system includes a light source that emits light including the light having the wavelength within the wavelength band, and the light guide plate guides at least the light having the wavelength within the wavelength band out of the light emitted from the light source, and causes the light within the wavelength band to be incident on each of the plurality of sites.
(20) The biological sensor according to (19), in which the light guide plate is transparent with respect to light having a wavelength within the wavelength band.
(21) The biological sensor according to (19) or (20), in which
   the light guide plate includes a plurality of diffraction units respectively corresponding to the plurality of light receiving elements and arranged on a side of the optical system, and
   each of the plurality of diffraction units diffracts the incident light having the wavelength within the wavelength band toward a corresponding site among the sites.
(22) The biological sensor according to any one of (11) to (21), further including a circularly polarizing plate arranged between the light receiving element array and the living body.
(23) The biological sensor according to any one of (1) to (22), in which the condition of the living body is a mental condition estimated from a sweating state of each of the sites.
(24) The present technology provides a biological condition determination method including:
   a step of irradiating a living body with irradiation light including light having a wavelength within a wavelength band including a peak wavelength related to absorption of water;
   a step of outputting signals of a plurality of sites by individually receiving the light having the wavelength within the wavelength band out of light emitted from the irradiation system and reflected from each of a plurality of sites of the living body; and
   a step of determining a condition of the living body on the basis of statistics related to the signals of the respective sites output in the outputting step.

### REFERENCE SIGNS LIST

10-1 to 10-23 Biological sensor
100-1, 100-4, 100-5, 100-11, 100-13 Irradiation system
110a, 110b Light source
200-1, 200-2, 200-3, 200-4, 200-6, 200-7, 200-8, 200-9,
200-10, 200-11, 200-12, 200-13, 200-19, 200-20, 200-21,
200-22, 200-23 Light receiving system
210, 210-11, 210-13 Light receiving element array
210a Light receiving element
220-1, 220-2, 220-3, 220-4, 220-6, 220-7, 220-8, 220-9,
220-10, 220-19, 220-20, 220-21, 220-22 Optical system
220a Lens array (optical member)
220a1 Light receiving lens
220b Wavelength selection filter
220c Circularly polarizing plate
220d Light shielding member (optical member)
220f Lens (optical member)
300-1, 300-2 Processing system
LB Living body
Each of plurality of sites LB1 to LB6
IL Irradiation light

## Claims

1. A biological sensor comprising:
an irradiation system that irradiates a living body with irradiation light including light having a wavelength within a wavelength band including a peak wavelength related to absorption of water;
a light receiving system that individually receives the light having the wavelength within the wavelength band out of light emitted from the irradiation system and reflected from each of a plurality of sites of the living body; and
a processing system that determines a condition of the living body on a basis of statistics related to signals of the respective sites output from the light receiving system.

2. The biological sensor according to claim 1, wherein the processing system determines the condition of the living body by using a first distribution that is a distribution of a number of signals for each of signal values of the signals of the respective sites.

3. The biological sensor according to claim 2, wherein the processing system determines the condition of the living body from a feature amount of the first distribution.

4. The biological sensor according to claim 3, wherein the feature amount of the first distribution is a number of signals whose signal values are equal to or less than a reference value of the signals of the respective sites.

5. The biological sensor according to claim 4, wherein the reference value is an average value of signal values of the signals of the respective sites.

6. The biological sensor according to claim 3, wherein the feature amount of the first distribution is a median value, an average value, or a mode value of signal values of the signals of the respective sites.

7. The biological sensor according to claim 2, wherein the processing system determines the condition of the living body by further using a second distribution that is a distribution of a number of signals for each of signal values of signals of the respective sites according to an inherent reflectance distribution of the plurality of sites.

8. The biological sensor according to claim 7, wherein the processing system compares a feature amount of the first distribution with a feature amount of the second distribution to determine the condition of the living body.

9. The biological sensor according to claim 8, wherein the feature amount of the second distribution is a feature amount corresponding to the feature amount of the first distribution.

10. The biological sensor according to claim 8, wherein
the feature amount of the first distribution is a proportion of numbers of signals whose signal values respectively match a plurality of values among the signals of the respective sites in the first distribution, and
the feature amount of the second distribution is a proportion of numbers of signals whose signal values respectively match the plurality of values among the signals of the respective sites in the second distribution.

11. The biological sensor according to claim 1, wherein
the light receiving system includes:
a light receiving element array including a plurality of light receiving elements respectively corresponding to the plurality of sites; and
an optical system corresponding to the plurality of light receiving elements, and
the optical system guides the light having the wavelength within the wavelength band out of the light emitted from the irradiation system and reflected from each of the plurality of sites to the light receiving element corresponding to the site.

12. The biological sensor according to claim 11, wherein
the irradiation light includes only the light having the wavelength within the wavelength band, and
the optical system includes an optical member that guides the light having the wavelength within the wavelength band, which has been emitted from the irradiation system and reflected by a corresponding site among the sites, to a corresponding light receiving element among the light receiving elements.

13. The biological sensor according to claim 11, wherein
the irradiation light includes the light having the wavelength within the wavelength band and light having a wavelength outside the wavelength band, and
the optical system includes a wavelength selection filter that selectively passes the light having the wavelength within the wavelength band out of the light emitted from the irradiation system and reflected from each of the plurality of sites.

14. The biological sensor according to claim 13, wherein the optical system includes an optical member that guides the light having the wavelength within the wavelength band, which has passed through the wavelength selection filter, to a corresponding light receiving element among the light receiving elements.

15. The biological sensor according to claim 13, wherein the optical system includes an optical member that guides the light having the wavelength within the wavelength band to a corresponding light receiving element among the light receiving elements via the wavelength selection filter.

16. The biological sensor of claim 11, wherein
the irradiation system includes:
a light source that emits the light having the wavelength within the wavelength band and light having a wavelength outside the wavelength band; and
a wavelength selection filter that selectively passes the light within the wavelength band out of the light within the wavelength band and the light outside the wavelength band emitted from the light source.

17. The biological sensor according to claim 16, wherein the optical system includes an optical member that guides the light having the wavelength within the wavelength band, which has passed through the wavelength selection filter and been reflected by a corresponding site among the sites, to a corresponding light receiving element among the light receiving elements.

18. The biological sensor according to claim 11, wherein the irradiation system includes at least one light source that is arranged between a pair of the light receiving elements adjacent to each other among the plurality of light receiving elements and emits light including the light having the wavelength within the wavelength band.

19. The biological sensor according to claim 11, further comprising
a light guide plate arranged between the optical system and the living body, wherein
the irradiation system includes a light source that emits light including the light having the wavelength within the wavelength band, and
the light guide plate guides at least the light having the wavelength within the wavelength band out of the light emitted from the light source, and causes the light within the wavelength band to be incident on each of the plurality of sites.

20. The biological sensor according to claim 19, wherein the light guide plate is transparent with respect to light having a wavelength within the wavelength band.

21. The biological sensor according to claim 19, wherein
the light guide plate includes a plurality of diffraction units respectively corresponding to the plurality of light receiving elements and arranged on a side of the optical system, and
each of the plurality of diffraction units diffracts the incident light having the wavelength within the wavelength band toward a corresponding site among the sites.

22. The biological sensor according to claim 11, further comprising a circularly polarizing plate arranged between the light receiving element array and the living body.

23. The biological sensor according to claim 1, wherein the condition of the living body is a mental condition estimated from a sweating state of each of the sites.

24. A biological condition determination method comprising:
a step of irradiating a living body with irradiation light including light having a wavelength within a wavelength band including a peak wavelength related to absorption of water;
a step of outputting signals of a plurality of sites by individually receiving the light having the wavelength within the wavelength band out of light emitted from the irradiation system and reflected from each of a plurality of sites of the living body; and
a step of determining a condition of the living body on a basis of statistics related to the signals of the respective sites output in the outputting step.
